# EUROPEAN PATENT APPLICATION

(11) **EP 1 418 238 A1**
(43) Date of publication of application: **12.05.2004**
(21) Application number: 02751628.5
(22) Date of filing: 18.07.2002
(51) Int. Cl.: C12Q 1/02, G01N 21/64, G01N 33/483, G01N 33/569, G01N 33/02, G01N 1/10, G01N 1/30, C12M 1/34

(54) **MICROBE EXAMINING DEVICE AND METHOD**

(30) Priority: 18.07.2001 JP 2001218625
(71) Applicant: ASAHI BREWERIES, LTD., Tokyo 104-0031 (JP); Leica Microsystems Kabushiki Kaisha, Shinagawa-ku, Tokyo 141-0032 (JP)
(72) Inventor: OGAWA, Akio, c/o LEICA MICROSYSTEMS KK, Shinagawa-ku, Tokyo 141-0032 (JP); YASUHARA, Takaomi, c/o ASAHI BREWERIES, LTD.,, Moriya-shi, Ibaraki 302-0106 (JP); MOTOYAMA, Yasuo, c/o ASAHI BREWERIES, LTD.,, Moriya-shi, Ibaraki 302-0106 (JP); TAKAHASHI, Kyoko, c/o ASAHI BREWERIES, LTD.,, Moriya-shi, Ibaraki 302-0106 (JP)
(74) Representative: Santarelli
(86) International application number: PCT/JP2002/007295
(87) International publication number: WO 2003/008634

(57) **Abstract**

An inspection equipment can be provided, which captures microbes in a sample solution with a filter, and detects information about microbes from a sample obtained by fluorescence-staining the microbes. The filter is irradiated with different excitation light beams (1, 2), and binary images (A, B, C) of fluorescences obtained in the respective fields in correspondence with the respective excitation light beams are sensed. The binary images (A, B, C) obtained with respect to the respective excitation light beams (1, 2) are compared to automatically identify the binary image B, which emits fluorescence with respect to only a specific excitation light beam (e.g., 1), as a fluorescent image based on a microbe, and the binary images A and C, which emit fluorescence with respect to all the excitation light beams, as fluorescent images other than those of microbes, thereby easily identifying microbes in the sample solution in an unmanned fashion.

## Description

### TECHNICAL FIELD

The present invention relates to a microbe inspection equipment and method and, more particularly, to a microbe inspection equipment and method which capture microbes and the like contained in, for example, a sample solution with a filter, fluorescent-stains the captured microbes, and automatically identify and display the microbe and others by using a microscope.

### BACKGROUND OF THE INVENTION

Conventionally, microbe tests in manufacturing process control, product quality control, and the like for beverages like beer, foods, medicines, cosmetics, and the like have been conducted by cultivation tests which require a large variety of culture media, and have taken many days to complete.

It has therefore taken much time to know test results, e.g., whether any microbes are present in a test target, the number of microbes, and identification of microbe species in the test target, resulting in placing large restrictions on the research, development, manufacturing, or shipping stages in various kinds of fields.

Under the circumstances, methods of quickly measuring microbes in liquid samples containing microbes have been proposed so far (e.g., "Current Trends in Microbe Testing Techniques", food processing and ingredients, 35(1), pp. 32 - 40 (2000)).

Known methods of quickly measuring microbes include, for example, an impedance method (Brown, D., Warner, M., Taylor, C., and Warren, R., Clin. Pthol., 37, 65 - 69 (1984)), an enzyme-fluorescence detection method (Japanese Patent Laid-Open No. 58-116700), a PCR method, a DEFT method as a combination of a membrane. filter method and a epifluorescent microscope method (G.L. PETTIPHER, UBALDINAM. RODRIGUES, J. Appl. Bacteriol. 53, 323 (1982)), and an RMDS method as a combination of a membrane filter method and an ATP method (Takahashi, T., Nakaita. Y., Watari. J., and Shinotsuka. K., Biosci. Biotechnol. Biochem. 64(5), pp. 1032 - 1037 (2000)). Devices adapting these measurement principles are commercially available.

The above methods, however, have many unsolved problems, e.g., 1) insufficient accuracy, 2) unsuitable for quick measurement, 3) insufficient quantitativeness, 4) high possibility of human errors, and 5) high running costs for culture media, reagents, and the like necessary for measurement.

Another known method is to separate microbes and the like by filtering the microbes existing in a sample solution, fluorescent-stain the sample containing the obtained microbes, and allow an observer to identify the microbes and others while visually observing the sample with a epifluorescent microscope.

In the above method, however, since the observer identifies microbes and others while visually observing fluorescent images generated from a fluorescence-stained sample, the measurement result may contain errors due to misidentification by the person who makes measurement. In addition, it has been impossible to identify microbes and others in an unmanned fashion.

### DISCLOSURE OF INVENTION

The present invention has been made to solve the above problems in the prior art, and has as its object to provide a microbe inspection equipment and method which can automatically and quickly identify microbes in a sample as a test target.

In order to achieve the above object, a testing method according to an embodiment of the present invention has the following arrangement. There is provided a testing method of testing a microbe contained in a sample, characterized by comprising an irradiation step of irradiating the sample with a plurality of excitation light beams having different wavelengths, and an identification step of identifying a microbe contained in the sample on the basis of a distribution of peaks of fluorescence obtained from each object contained in the sample in correspondence with irradiation with the plurality of excitation light beams.

In this case, for example, preferably, the method further comprises an inspection step of specifying a fluorescent object that can be a microbe on the basis of fluorescence intensities or shapes of fluorescent objects obtained from the respective objects, and in the identification step, a distribution of peaks of the fluorescence is obtained by using the fluorescent object specified in the inspection step.

In this case, for example, preferably, in the irradiation step, the sample is sequentially or simultaneously irradiated with the plurality of excitation light beams having different wavelengths.

In this case, for example, preferably, fluorescence obtained from each object contained in the sample has not less than one peak, and in the identification step, a microbe contained in the sample is identified on the basis of each peak wavelength or frequency of fluorescence obtained from each object contained in the sample.

In this case, for example, preferably, in the identification step, each peak wavelength or frequency of fluorescence obtained from each of the objects is collated with determination criteria defined in advance in correspondence with the plurality of excitation light beams to determine whether or not each of the objects is a microbe.

In this case, for example, the microbe is preferably a specific microbe.

In this case, for example, preferably, fluorescence obtained from each object contained in the sample has not less than one peak, and in the identification step, a microbe contained in the sample is identified on the basis of a fluorescence spectrum obtained from each object contained in the sample.

In this case, for example, preferably, in the identification step, a fluorescence spectrum obtained from each of the object is collated with determination fluorescence spectra defined in advance in correspondence with the plurality of excitation light beams to determine whether or not each of the objects is a microbe.

In this case, for example, the microbe is preferably a specific microbe.

In this case, for example, preferably, the testing method comprises a primary inspection step, and a secondary inspection step, in the primary inspection step, a fluorescent object contained in the sample is specified by observing an entire region of the sample at a first magnification while irradiating the sample with the excitation light, and in the secondary inspection step, the irradiation step and the identification step are executed, and a distribution of peaks of fluorescence from each of the fluorescent objects is obtained while each fluorescent object specified in the primary inspection step is observed at a second magnification higher than the first magnification.

In this case, for example, preferably, the testing method comprises a primary inspection step, and a secondary inspection step, in the primary inspection step, a target microbe is separated from microbes other than the target microbe among fluorescent objects contained in the sample by observing an entire region of the sample at a first magnification while irradiating the sample with the excitation light, and in the secondary inspection step, the irradiation step and the identification step are executed, and a distribution of peaks of fluorescence from each of the target microbes is obtained while each target microbe extracted in the primary inspection step is observed at a second magnification higher than the first magnification.

In this case, for example, preferably, the method further comprises a sample preparation step of capturing a microbe contained in the sample on a filter, and staining objects including the microbe captured on the filter with a fluorescent dye.

In this case, for example, preferably, the method further comprises a sample preparation step of capturing a microbe contained in the sample on a filter, and staining the microbe captured on the filter with a fluorescent dye such that the microbe captured on the filter has a peak in not less than one fluorescence upon irradiation of excitation light including not less than two wavelengths.

In this case, for example, as the excitation light including not less than two wavelengths, not less than two excitation light beams selected from excitation light beams having wavelengths falling within a range of 340 nm to 750 nm at maximum intensities are preferably used.

In this case, for example, as the fluorescent dye, not less than one fluorescent dye selected from the group consisting of Texas Red, tetramethylrhodamine, indo-carbocyanine dye, Alexa dye, 4',6-diamidino-2-phenylindole (DAPI), providium iodide, and fluorescein isothiocyanate (FITC) is preferably used.

In this case, for example, preferably, the method further comprises a sample preparation step of capturing a microbe contained in the sample on a filter, and staining the microbe captured on the filter with different kinds of fluorescent dyes such that the microbe captured on the filter has a peak in not less than two fluorescences upon irradiation of excitation light including not less than three wavelengths.

In this case, for example, as the excitation light including not less than three wavelengths, not less than three excitation light beams selected from excitation light beams having wavelengths falling within a range of 340 nm to 750 nm at maximum intensities are preferably used.

In this case, for example, as the fluorescent dye, not less than two fluorescent dye selected from the group consisting of Texas Red, tetramethylrhodamine, indo-carbocyanine dye, Alexa dye, 4',6-diamidino-2-phenylindole (DAPI), providium iodide, and fluorescein isothiocyanate (FITC) are preferably used.

In order to achieve the above object, an inspection equipment according to an embodiment of the present invention has the following arrangement. There is provided an inspection equipment for testing a microbe contained in a sample, characterized by comprising an irradiation mechanism which irradiates the sample with a plurality of excitation light beams having different wavelengths, an image sensing device which image-senses the sample, and an analyzing device which analyzes an image sensing result obtained by the image sensing device, wherein the analyzing device is configured to identify, on the basis of the image sensing result, a microbe contained in the sample on the basis of a distribution of peaks of fluorescence obtained from each object contained in the sample in correspondence with irradiation with the plurality of excitation light beams.

In this case, for example, preferably, the analyzing device further comprises a testing unit which specifies a fluorescent object that can be a microbe on the basis of fluorescence intensities or shapes of fluorescent objects obtained from the respective objects, and the analyzing device obtains a distribution of peaks of the fluorescence by using the fluorescent object specified by the testing unit.

In order to achieve the above object, an inspection equipment according to an embodiment of the present invention has the following arrangement. There is provided an inspection equipment for testing a microbe contained in a sample, characterized by comprising an input device which receives a result obtained by image-sensing the sample while irradiating the sample with a plurality of excitation light beams having different wavelengths, and an analyzing device which identifies a microbe contained in the sample on the basis of a distribution of peaks of fluorescence obtained from each object contained in the sample in correspondence with the plurality of excitation light beams in accordance with the image sensing result received by the input device.

In this case, for example, preferably, the irradiation mechanism sequentially or simultaneously irradiates the sample with the plurality of excitation light beams having different wavelengths.

In this case, for example, preferably, fluorescence obtained from each object contained in the sample has not less than one peak, and the analyzing device identifies a microbe contained in the sample on the basis of each peak wavelength or frequency of fluorescence obtained from each object contained in the sample.

In this case, for example, the analyzing device preferably collates each peak wavelength or frequency of fluorescence obtained from each of the objects with determination criteria defined in advance in correspondence with the plurality of excitation light beams to determine whether or not each of the objects is a microbe.

In this case, for example, preferably, in the inspection equipment, fluorescence obtained from each object contained in the sample has not less than one peak, and the analyzing device identifies a microbe contained in the sample on the basis of a fluorescence spectrum obtained from each object contained in the sample.

In this case, for example, the analyzing device preferably collates a fluorescence spectrum obtained from each of the object with determination fluorescence spectra defined in advance in correspondence with the plurality of excitation light beams to determine whether or not each of the objects is a microbe.

In this case, for example, preferably, the inspection equipment further comprises a control device which controls the irradiation mechanism and the image sensing device, the control device performing control to image-sense an entire region of the sample by using the image sensing device at a first magnification while irradiating the sample with the excitation light by using the irradiation mechanism and specify a fluorescent object contained in the sample by analyzing an image sensing result obtained by the image sensing device by using the analyzing device, and then performing control to image-sense only each of the specified fluorescent objects by using the image sensing device at a second magnification higher than the first magnification while irradiating each of the specified fluorescent objects with the excitation light by using the irradiation mechanism and obtain a distribution of peaks of fluorescence from each of the fluorescent dyes by analyzing an image sensing result obtained by the image sensing device by using the analyzing device.

In this case, for example, preferably, the testing further comprises a control device which controls the irradiation mechanism, the image sensing device, and the analyzing device, the control device performing control to image-sense an entire region of the sample by using the image sensing device at a first magnification while irradiating the sample with the excitation light by using the irradiation mechanism and extract a target microbe, among fluorescent objects contained in the sample, while separating the target microbe from a microbe other than the target microbe by analyzing an image sensing result obtained by the image sensing device by using the analyzing device, and performing control to image-sense each of the extracted target microbes by using the image sensing device at a second magnification higher than the first magnification and obtain a distribution of peaks of fluorescence from the target microbe by analyzing an image sensing result obtained by the image sensing device by using the analyzing device.

In this case, for example, as the plurality of excitation light beams, not less than two excitation light beams selected from excitation light beams having wavelengths falling within a range of 340 nm to 750 nm at maximum intensities are preferably used.

In this case, for example, preferably, the image sensing device further comprises a motor-driven stage, and the control device controls the image sensing device to image-sense an entire region of the sample while controlling the motor-driven stage to scan the entire region of the sample.

In this case, for example, preferably, the image sensing device comprises an objective lens and an equation which is set in advance to keep a distance between the objective lens and a surface of the filter constant, and the control device controls the image sensing device on the basis of the equation to image-sense the entire region of the sample while keeping the distance between the objective lens and the surface of the filter constant so as not to cause an out-of-focus state during the scanning.

In order to achieve the above object, a control program according to an embodiment of the present invention has the following arrangement. There is provided a control program which controls an inspection equipment for testing a microbe contained in a sample, characterized by comprising an identification step of, when the inspection equipment irradiates the sample with a plurality of excitation light beams having different wavelengths, identifying a microbe contained in the sample on the basis of a distribution of peaks of fluorescence obtained from each object contained in the sample in correspondence with irradiation with the plurality of excitation light beams.

In order to achieve the above object, a computer-readable storage medium according to an embodiment of the present invention has the following arrangement. There is provided a computer-readable storage medium storing a control program which controls an inspection equipment for testing a microbe contained in a sample, characterized in that the control program comprises an identification step of, when the inspection equipment irradiates the sample with a plurality of excitation light beams having different wavelengths, identifying a microbe contained in the sample on the basis of a distribution of peaks of fluorescence obtained from each object contained in the sample in correspondence with irradiation with the plurality of excitation light beams.

The microbe testing method and equipment having the above arrangements irradiate a sample with excitation light beams of two or three or more wavelengths, and compare a plurality of fluorescent images obtained in correspondence with the respective excitation light beams, thereby automatically identifying microbes contaminated in a sample solution. This makes it possible to shorten the testing time and prevent measurement errors due to human errors.

### BRIEF DESCRIPTION OF DRAWINGS

Fig. 1 is a diagram showing a microbe inspection equipment according to an embodiment of the present invention;
Fig. 2 is a diagram for explaining the overall arrangement of the microbe inspection equipment according to an embodiment of the present invention;
Fig. 3 is a diagram showing the relationship between fluorescent dyes, excitation light beams, and fluorescences;
Fig. 4 is a diagram for explaining a concatenated component;
Fig. 5 is a flow chart showing primary automatic identification processing of extracting the location of a fluorescent object on the entire region of a sample;
Fig. 6 is a flow chart for secondary automatic identification processing of extracting a microbe by a 1-wavelength identification method;
Fig. 7 is a flow chart for secondary automatic identification processing of extracting a microbe by a 2-wavelength identification method;
Fig. 8 is a diagram for explaining microbe identification methods and their determination criteria;
Fig. 9 is a diagram for explaining methods of calculating a curve length, curve width, and roundness from a concatenated component of pixels;
Fig. 10 is a diagram for explaining examples in which curve lengths, curve widths, and roundnesses are calculated from concatenated components of pixels;
Fig. 11 is a diagram for explaining an example of binary images in the respective fields which are obtained by secondary automatic identification processing in the 2-wavelength identification method;
Fig. 12 is a diagram for explaining a sequence for obtaining fluorescence spectra (or peak wavelengths) from the binary images in the respective fields by the 2-wavelength identification method;
Fig. 13 is a diagram showing an example of determination fluorescence spectra or determination criteria used in the 2-wavelength identification method;
Fig. 14 is a flow chart for processing of identifying a microbe from a fluorescent object in each field;
Fig. 15 is a flow chart for secondary automatic identification processing of identifying a microbe by a "3 or more" wavelength identification method;
Fig. 16 is a diagram for explaining an example of binary images in the respective fields which are obtained by primary automatic identification processing and secondary automatic identification processing in the 3-wavelength identification method;
Fig. 17 is a diagram for explaining a sequence for obtaining fluorescence spectra from binary images in the respective fields which are obtained by secondary automatic identification processing in an example of the 3-wavelength identification method;
Fig. 18 is a diagram showing an example of determination fluorescence spectra or determination criteria used in the 3-wavelength identification method;
Fig. 19 is a diagram for explaining another example of binary images in the respective fields which are obtained by primary automatic identification processing and secondary automatic identification processing in the 3-wavelength identification method; and
Fig. 20 is a diagram showing another example of determination fluorescence spectra used in the 3-wavelength identification method in Fig. 19.

### BEST MODE FOR CARRYING OUT THE INVENTION

A preferred embodiment of the present invention will be described below with reference to the accompanying drawings.

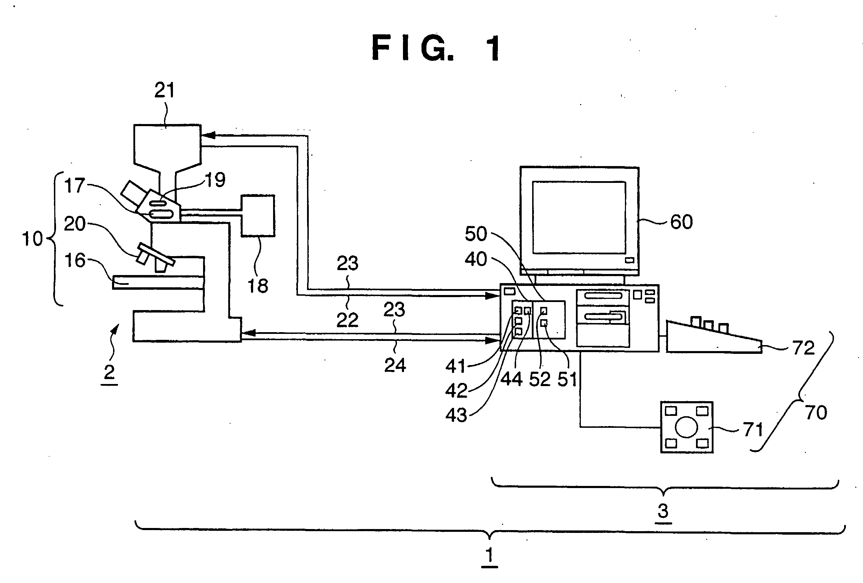

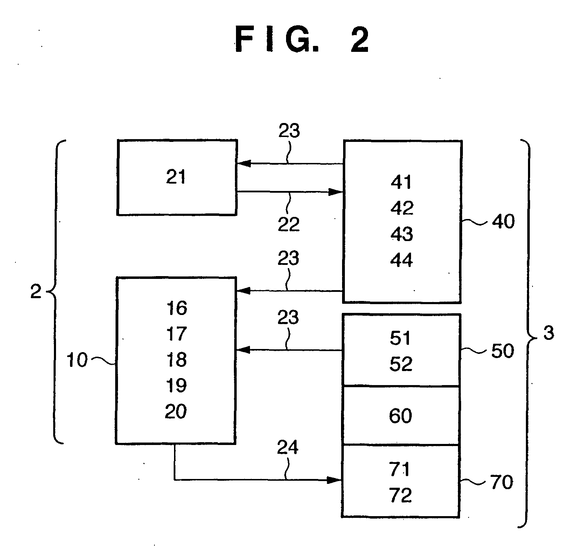

### [Microbe Inspection Equipment: Figs. 1 and 2]

A microbe inspection equipment 1 according to an embodiment of the present invention will be described below with reference to Figs. 1 and 2. Fig. 1 shows a outline for explaining the overall arrangement of the microbe inspection equipment 1. Fig. 2 shows a outline for explaining control on each component of the microbe inspection equipment 1.

Referring to Fig. 1, the microbe inspection equipment 1 is comprised of a epifluorescent microscope 2 and image analyzing unit 3.

The epifluorescent microscope 2 has a epifluorescent microscope body 10 which magnifies a sample containing microbes for observation and an image capturing unit 21 (e.g., a monochrome or color camera such as a cooled CCD camera) for photoelectrically converting the magnified image. The image capturing unit 21 is controlled (23) by the image analyzing unit 3. Image data 22 obtained by the image capturing unit 21 is transmitted to the image analyzing unit 3. A computing unit 50 and identifying unit 44 analyze the image data 22 to identify microbes contained in the sample.

A sample containing microbes is, for example, a beverage such as beer, which basically should contain no microbes or debris other than microbes (test sample solution). In this case, however, a sample containing microbes is a sample extracted from a test sample solution in a predetermined amount to check in the manufacturing process or the like whether microbes or debris other than microbes are contaminated in the solution. A sample extracted from a test sample solution to be used in the microbe inspection equipment 1 is prepared in the following steps: filtering the test sample solution with a filtering unit using a membrane filter, capturing microbes and debris other than microbes on the membrane filter, removing the membrane filter from the filtering unit, and applying fluorescent dyes to the membrane filter which has captured microbes and others to stain the microbes in the sample with the fluorescent dyes. Note that one or a plurality of fluorescent dyes are used to stain microbes in a sample, as needed.

The optical microscope 10 includes a microscope motor-driven stage 16 on which a sample containing fluorescence-stained microbes is to be placed, an electric focus motor 17, a light source unit 18 which intensely fluorescence-labels a target microbe by irradiating the sample with excitation light emitted from a high-output mercury lamp, xenon lamp, or the like, a fluorescence filter block switching unit 19 having filters which are placed in an optical path from the light source unit 18 to the microscope motor-driven stage 16 to select one or a plurality of specific wavelengths of those of excitation light beams and select one or a plurality of specific wavelengths of those of fluorescences emitted from the sample upon irradiation with the excitation light, and a lens switching unit 20 which switches objective lenses.

In order to excite the sample containing microbes stained with fluorescent dyes, the optical microscope 10 sequentially irradiates the sample with a plurality of excitation light beams having different specific wavelengths. The optical microscope 10 can detect the respective fluorescences obtained in accordance with the respective excitation light beams by sequentially switching the filters of the fluorescence filter block switching unit 19. Alternatively, the optical microscope 10 may simultaneously irradiate the sample with a plurality of excitation light beams having different specific wavelengths.

The optical microscope 10 sends measurement information 24 including current conditions, e.g., a lens, filter, stage position, and focus position, to the image analyzing unit 3.

The image analyzing unit 3 includes a control unit 40 which executes computation necessary for control on the electric focus motor 17 for the microscope motor-driven stage 16, the computing unit 50 which performs appropriate processing for the obtained image data 22 to automatically calculate feature information for identification of a microbe on the basis of each concatenated component of pixels (to be described later), an input unit 70 constituted by a keyboard 72 which inputs the definition of an inspection manner, a limit value used for determination on a microbe, and the like, a trackball 71 for stage focus movement, and the like, and a display unit 60 which displays the image-sensing result, various analysis results, and the like obtained by the optical microscope.

Note that since the image analyzing unit 3 performs processing for each excitation light, the fluorescent images emitted from fluorescent objects in the respective areas can be acquired by using excitation light beams having different specific wavelengths. When fluorescent images are acquired, they are stored in correspondence with the respective excitation light beams. In addition, the fluorescent images obtained by the respective excitation light beams are combined to form a fluorescence spectrum (or a peak wavelength). This fluorescence spectrum is then compared with a predetermined determination fluorescence spectrum (or determination criterion). This makes it possible to identify a target microbe, microbes other than the target, debris other than microbes, and the like. The determination fluorescence spectrum (or determination criterion) is stored in the image analyzing unit 3.

The image analyzing unit 3 has an automatic fluorescence inspection function for controlling the respective steps from the measurement of a sample to analysis for identifying a microbe. This automatic fluorescence inspection function is executed by the CPU of the image analyzing unit 3 by using a RAM on the basis of the automatic fluorescence inspection program stored in the ROM of the image analyzing unit 3. The function includes a function of driving the microscope motor-driven stage 16 to scan the entire surface of a fluorescence-stained sample (e.g., a sample obtained by capturing a microbe on a membrane filter and fluorescence-staining it), a focus control function executed for each measurement visual field in synchronism with scanning on the entire surface of a sample, a function of storing a location in a sample from which a fluorescence signal is detected and allowing reconfirmation of the location by microscopic observation of a fluorescent object in the region after scanning on the sample (for example, a method of using a lens with a higher magnification than that in a primary entire scanning test, a method of applying one or more different excitation light beams in addition to excitation light used in a primary test, or a combination thereof, i.e., unmanned, automatic, visual Validation function), a function of automatically detecting a feature amount such as a fluorescence intensity or shape from each concatenated component in a captured image, and specifying a fluorescent object that can be a microbe, a function of automatically generating a fluorescence spectrum or peak wavelength on the basis of the specified fluorescent object that can be a microbe, and identifying the microbe, and the like.

The control unit 40 includes an motor-driven focus control unit 41 which always obtains correct focus by executing focus control following the movement of the microscope motor-driven stage 16 which moves a sample base for each predetermined area of a membrane filter, whose entire area is divided into predetermined areas, to sequentially irradiate the entire area of the membrane filter with excitation light, a motor-driven focus control unit 42 which drives the microscope motor-driven stage 16 to scan the entire surface of a sample containing microbes, a microscope/camera control unit 43, and an identifying unit 44 which identifies a microbe on the basis of the image data 22 transmitted from the image capturing unit 21. The microscope/camera control unit 43 controls a light source shutter, lens switching, fluorescence filter block switching, exposure start timing, exposure time, and the like. Various kinds of control can be performed by using the control unit 40. For example, the following control can be done: setting the optical microscope 10 to a low magnification by using a low-power lens, detecting and storing fluorescent objects by scanning the entire surface of a sample containing microbes while sequentially irradiating each region of the sample with excitation light having a specific wavelength (primary automatic identification), and precisely identifying the respective fluorescent objects while sequentially irradiating only regions, from which the fluorescent objects have been detected, with excitation light beams having one or two or more different specific wavelengths by using a high-power lens.

The computing unit 50, which calculates a sample scanning region count in a domain of a fluorescent object to be measured which has a maximum diameter, includes an inspection region definition computing unit 51 and a predictive focus computing unit 52 which controls the microscope motor-driven stage 16 and electric focus motor 17 to set a focal point on a predetermined plane of a sample containing microbes in scanning the entire surface of the sample and always automatically achieve focus on the same plane (this method is sometimes referred to as a predictive focus method or the like).

The predictive focus method will be described in more detail. The predictive focus method is a method of setting in advance an equation (sample plane equation) for keeping the distance between a sample surface and an objective lens constant (constant in the Z direction) within a defined scanning range (a predetermined range in the X and Y directions) and automatically controlling a focal position according to the sample plane equation in accordance with scanning coordinates during measurement scanning.

### [Microbe Inspection Method]

A method of automatically identifying microbes by using the above microbe inspection equipment 1 will be described next.

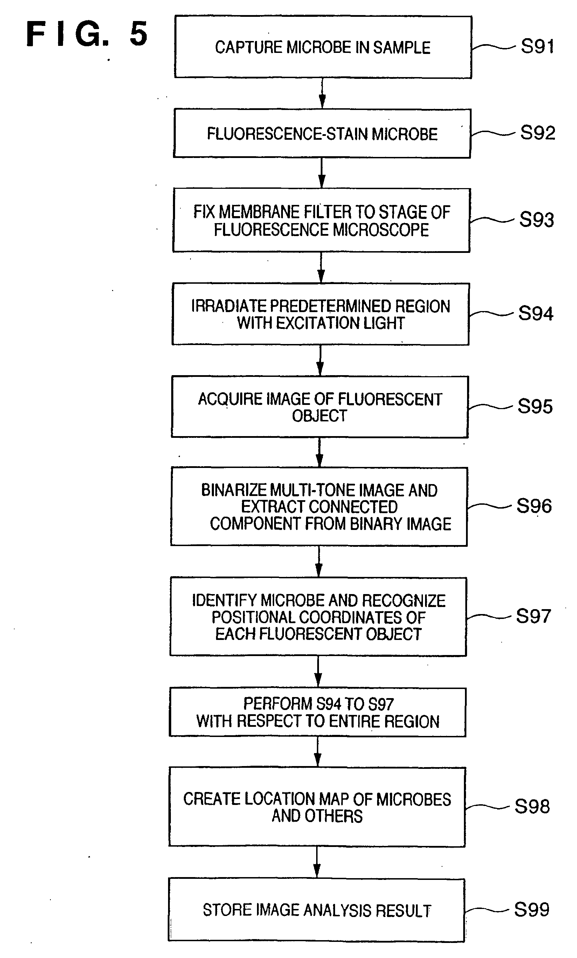

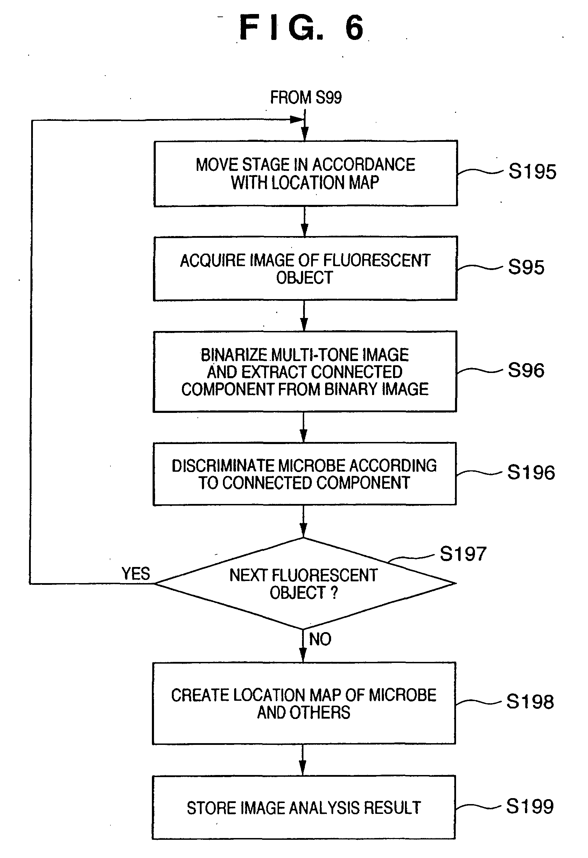

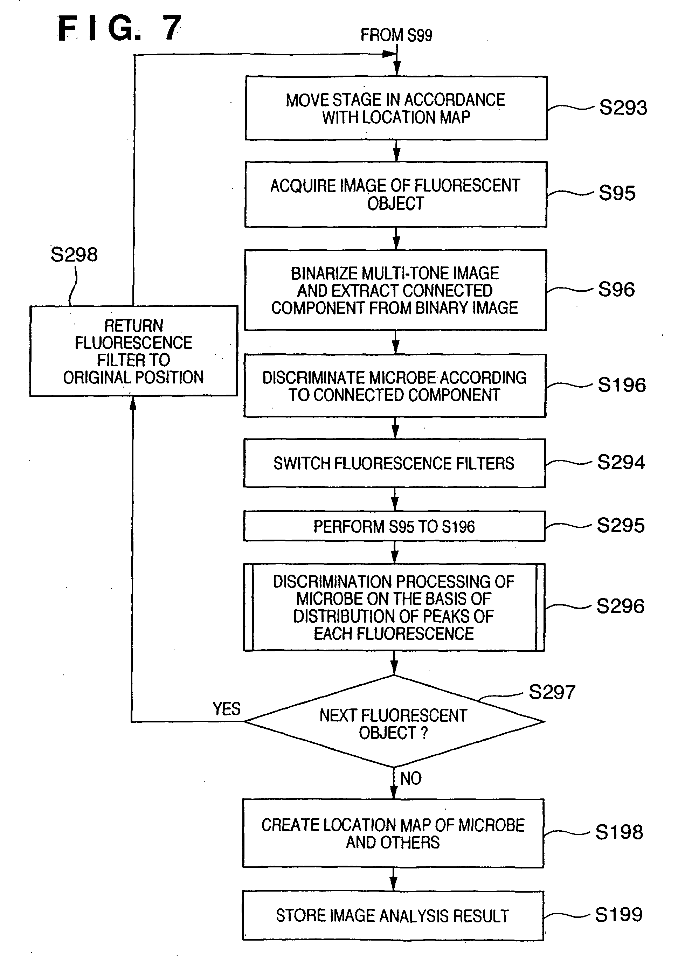

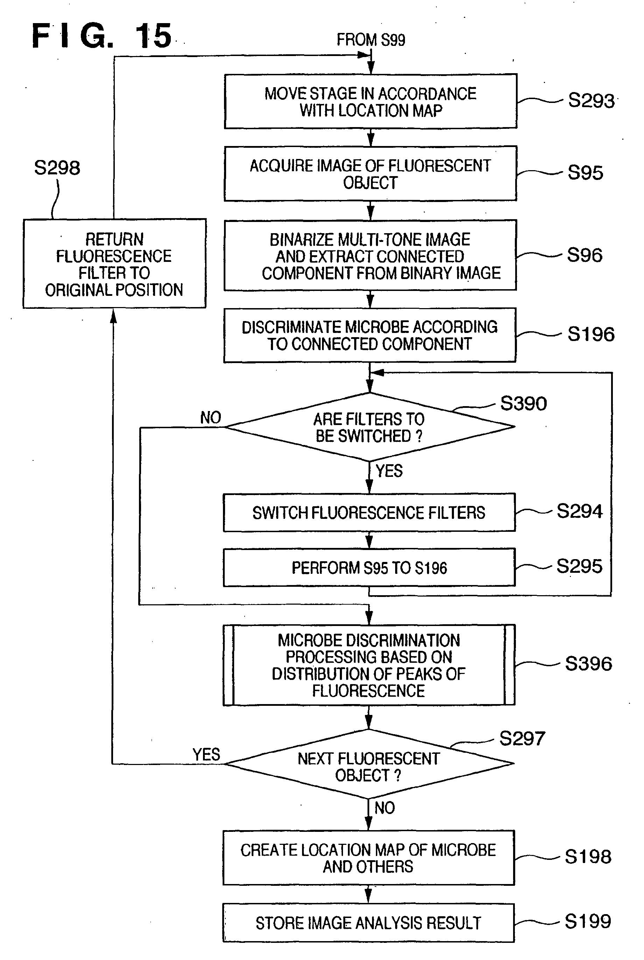

Primary automatic identification processing of extracting the locations of fluorescent objects from the entire region of a sample will be described first with reference to Fig. 5. With regard to, secondary automatic identification processing of identifying microbes from the fluorescent objects extracted by the primary automatic identification processing, three kinds of identifying methods, i.e., a 1-wavelength identifying method (Fig. 6), 2-wavelength identifying method (Fig. 7), and 3-wavelength identifying method (Fig. 15), will be described in detail below.

Primary automatic identification processing and secondary automatic identification processing can be performed by using a lens with any magnification. Assume, however, that in the following description, primary automatic identification processing is performed by using a low-power lens, and secondary automatic identification processing is performed by using high-power lens.

### [Outline of Primary Automatic Identification Processing: Fig. 5]

Fig. 5 is a flow chart for explaining the steps in primary automatic identification processing for extracting the locations of fluorescent objects from the entire region of a sample.

Note that steps S91 to S93 correspond to preprocessing, and steps S94 to S99 correspond to primary automatic identification processing. This primary automatic identification processing is executed by the image analyzing unit 3 on the basis of an automatic fluorescence inspection program.

In step S91, a test sample solution is filtered by a filtering unit using a membrane filter having a predetermined filter diameter, e.g., 10 to 50 mm, to capture microbes and debris other than microbes on the membrane filter.

In step S92, the microbes captured on the membrane filter are stained with predetermined fluorescent dyes. For example, as this staining method, a FISH method, fluorescent antibody method, nucleic acid staining method, or enzymatic staining method is available.

In step S93, the membrane filter containing the stained sample is placed to the microscope motor-driven stage 16 of the microbe inspection equipment 1. This completes preparation of the sample from the test sample solution.

In step S94, the membrane filter containing the stained sample which is placed to the microscope motor-driven stage 16 is irradiated with excitation light beams having specific wavelengths corresponding to the respective fluorescent dyes. Note that the membrane filter which is to be irradiated with excitation light beams is divided in advance into areas each having a predetermined size, and the filter is irradiated with excitation light for each divided area.

In step S95, fluorescent images having specific wavelengths are captured, which are emitted from portions of the sample in which the respective fluorescent dyes are absorbed by microbes, in accordance with the applied excitation light beams.

In step S96, 1-bit gray-level binary image data is acquired from the obtained fluorescent images by using a binarization method, thereby extracting concatenated components necessary for identification processing of microbes. Alternatively, proper image processing may be performed for the obtained fluorescent images to acquire 1-bit gray-level binary image data from the images after the image processing by using the binarization method, thereby extracting concatenated components necessary for identification processing of microbes.

In step S97, image analysis processing is performed to identify microbes and others, and the locations of the fluorescent objects formed from the respective concatenated components are determined. This series of steps for one region, i.e., from irradiation with excitation light beams in step S94 to identification processing of microbes in step S97, is performed for each region of the sample, and all the regions of the sample are scanned to determine the locations of fluorescent objects in the respective regions and perform primary determination of determining whether or not the fluorescent objects are microbes.

In step S98, the location map of microbes and others is generated, and detected microbes are displayed on the display screen. Microbes and others can be displayed on the display screen by three kinds of discrimination methods.

In step S99, the image analysis result is stored.

### [Test Sample Solution: Fig. 5]

Primary automatic identification processing in Fig. 5 will be described in detail next. The primary automatic identification processing is performed by using a low-power lens.

A test sample solution to be measured by the microbe inspection equipment 1 is, for example, a beverage such as beer, which basically should contain no microbes or debris other than microbes.

In a manufacturing processing or the like, however, microbes or debris other than microbes may be contaminated in a sample. Microbes in a beverage include, for example, bacteria and yeasts. For example, Pectinatus species which is a bacteria harmful to beer has a width of 0.5 to 2 µm and a curve length of 1.5 to 10 µm. Another example is a yeast whose width and curve length fall within 3 to 10 µm. As described above, targets which are contaminated in a beverage may vary in size. For this reason, filters having different pore sizes can be selectively used in the microbe inspection equipment 1 in accordance with the size of a target which may be contaminated in a beverage.

Part of a beverage is sampled as a test sample solution at the correct time and is analyzed by using the microbe inspection equipment 1 described above. This makes it possible to automatically discriminate quickly and quantitatively whether or not microbes and debris other than microbes are contaminated in the beverage and to separately display the microbes and the debris other than microbes, thereby performing quality control on the beverage.

### [Preparation of Sample from Test Sample Solution: Steps S91 and S92 in Fig. 5]

In order to quantitatively analyze microbes in a test sample solution by using the microbe inspection equipment 1, sample preparation is performed in the following step.

First of all, a predetermined amount of test sample solution is sampled from a beverage such as beer. The test sample solution is then filtered with a filtering unit using a membrane filter to capture, on the membrane filter, all the microbes and debris other than microbes contained in the test sample solution.

The number of all microbes and debris other than microbes contained in the test sample solution can be quantitatively analyzed by counting the total number of microbes captured on the membrane filter by using the microbe inspection equipment 1 (this operation will be described in detail later).

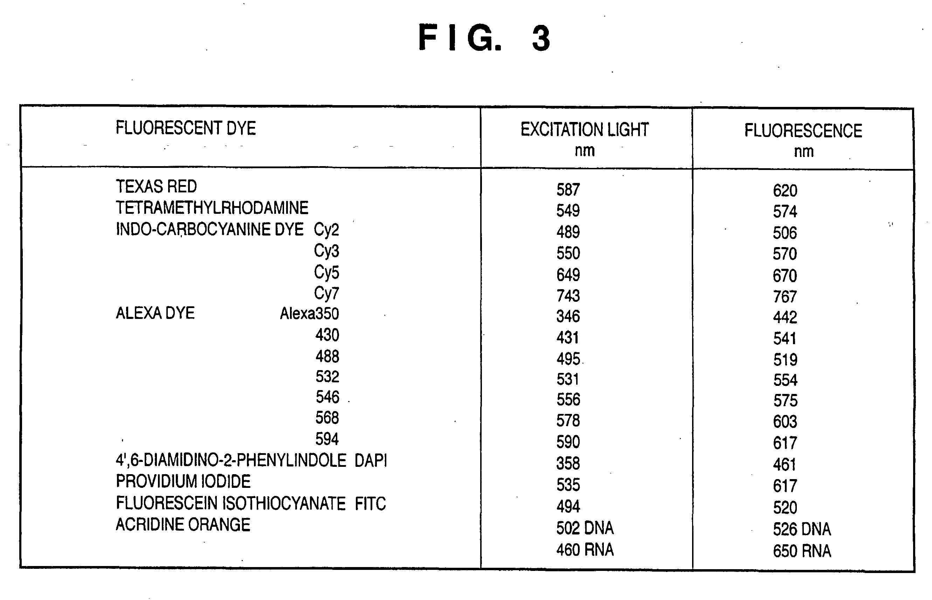

The membrane filter is then removed from the filtering unit. By applying fluorescent dyes to the microbes and others (to be referred to as a sample hereinafter), the microbes in the sample are stained with the fluorescent dyes. In this case, the microbes in the sample are stained with, for example, one or a plurality of kinds of fluorescent dyes selected from Fig. 3. When the membrane filter containing the stained sample is placed on the microscope motor-driven stage 16 of the microbe inspection equipment 1, preparation of the sample from the test sample solution is complete.

### [Membrane Filter: Step S91 in Fig. 5]

The above membrane filter will be described. The membrane filter has, for example, a flat shape like a disc with many pores. The filter diameter is about 10 to 50 mm, and the filter pore diameter is 0.2 to 50 µm. The number of pores of the filter can be arbitrarily optimized as needed. Using the membrane filter therefore makes it possible to capture microbes larger than the filter pore size.

### [Staining Method Using Fluorescent Dyes: Step S92 in Fig. 5]

A method of staining microbes captured on the above membrane filter with fluorescent dyes will be described in detail next. As a method of staining microbes with fluorescent dyes, the FISH method, fluorescent antibody method, or the like is available.

The FISH method will be described first. The FISH method is a method of fluorescence-staining a microbe by using a nucleic acid probe and targeting a nucleic acid in a cell. This method does not require the step of extracting a nucleic acid from a microbe, and directly adds a fluorescence-labeled nucleic acid probe to a pretreated microbe to make the probe hybridize to an rRNA or chromosome DNA of a nucleic acid in a microbial cell.

In general, an rRNA of a nucleic acid in a microbial cell is used as a probe target. There are several thousand to several hundred thousand rRNA copies in a microbial cell, and hence there are probe targets equal in number to the rRNA copies. For this reason, a large amount of fluorescent dye bonded to the nucleic acid probe is accumulated in the target microbial cell. When the fluorescent dye used in this case is irradiated with proper excitation light, only the target microbial cell emits fluorescence without changing its shape to allow its observation under the epifluorescent microscope.

In addition, the complementary sequence of strain specific region in a chromosome DNA can be used as a probe. Likewise, a microbial cell can be fluorescence-stained in a species-specific manner.

The fluorescent antibody method will be described next. The fluorescent antibody method is a method of selectively staining a target microbe by using an antibody which specifically recognizes an antigen constituted by the proteins, saccharides, lipid, or the like of a target microbial cell. This method uses an antibody which recognizes an antigen existing in the surface layer of a cell. By directly fluorescence-labeling an antibody or fluorescence-labeling a secondary antibody bonded to a primary antibody, a microbe having a surface antigen recognized by the primary antibody is specifically fluorescence-stained to be detected.

Fig. 3 shows an example of fluorescent dyes used when microbes captured on the above membrane filter are stained by using the FISH method or fluorescence antibody method. Fig. 3 shows the relationship between the fluorescent dyes, excitation light, and fluorescence.

Referring to Fig. 3, when each fluorescent dye is irradiated with excitation light having a specific wavelength corresponding to the fluorescent dye, the fluorescent dye emits fluorescence having a specific wavelength corresponding to the dye. Therefore, the use of Fig. 3 makes it possible to select a fluorescent dye, the wavelength of excitation light, and the wavelength of fluorescence light. Assume that indo-carbocyanine dye (Cy3) is selected as a fluorescent dye, and the dye is irradiated with excitation light having a wavelength of 550 nm. In this case, fluorescence having a wavelength of 570 nm can be observed. When a sample is stained with a plurality of fluorescent dyes in Fig. 3 and is irradiated with corresponding excitation light beams, a plurality of fluorescences having different wavelengths can be observed from the sample.

### [Analysis on Entire Surface of Sample and Extraction of Concatenated component: Step S95 in Fig. 5]

An analysis on the entire surface of a sample using the automatic fluorescence inspection function executed by the image analyzing unit 3 will be described next.

In executing the automatic fluorescence inspection function, a proper scanning range on the entire region of a sample is determined first. The magnification of an objective lens is set to, for example, 10x in accordance with the maximum-diameter domain of a fluorescent object as a measurement target, e.g., the range of 1 µm to 20 µm. A scanning step amount per frame (lateral direction: 1060 µm = 1100 - 20*2; longitudinal direction: 850 µm = 870 - 20) is automatically obtained from the effective visual field of the CCD camera which is uniquely determined by the above magnification, e.g., 1100 µm (in the lateral direction) x 870 µm (in the longitudinal direction).

In addition, the image analyzing unit 3 defines a measurement area, other than the image sensing area, per frame, and matches this value with the step amount. That is, settings are made such that adjacent camera image sensing range visual fields overlap each other by 20 µm on the two sides in the lateral direction and 20 µm on the upper side in the longitudinal direction per visual field in the camera image sensing range in scanning/image sensing operation.

Since a concatenated component having a concatenated component end point (the coordinates of the lowermost-rightmost pixel on a frame in the area occupied by the concatenated component) within the measurement area is set as a measurement target, the image analyzing unit 3 can reliably measure a target fluorescent object in just proportion by using the above setting method.

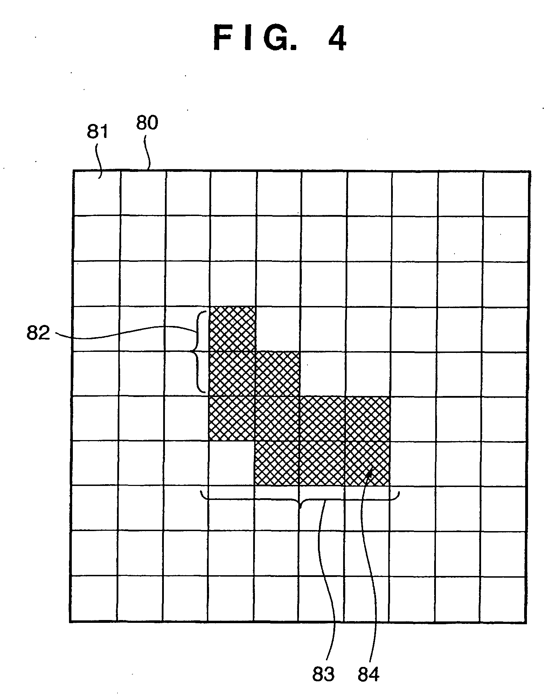

The above contents will be described in detail below with reference to a measurement area 80 on the frame shown in Fig. 4. Fig. 4 shows a binary image to be described later. Of pixels 81, "active" pixels having luminances equal to or higher than a predetermined luminance are displayed by hatching, and a "cluster" formed by connecting "active pixels" is defined as a concatenated component 82.

In addition, the area occupied by the concatenated component 82 shown on the central portion in Fig. 4 will be referred to as an occupied area 83 of the concatenated component; and the lowermost-rightmost pixel on the frame of the occupied area 83 of the concatenated component, a concatenated component end point 84.

The image analyzing unit 3 controls the microscope motor-driven stage 16 and electric focus motor 17 to always automatically focus on the same plane of a membrane filter on which a sample is captured. The absolute positional coordinates of an image of a target fluorescent object on the membrane filter are automatically determined from the positional coordinates of the controlled microscope motor-driven stage 16 (the coordinates of the camera image sensing range visual field) and the positional coordinates of a concatenated component measured on the frame.

This method can detect an image of a fluorescent object obtained on the entire region of a sample by unmanned automatic scanning operation. For example, whether or not each fluorescent object is a microbe can be automatically determined by setting in advance a limit value for microbe determination on the basis of a parameter such as the fluorescence intensity of an image of each fluorescent object or the feature value of the shape, e.g., an area, curve length, or curve width (to be described in detail later), and comparing each limit value with the above feature value obtained from one or a plurality of fluorescence intensity measurement results.

Since the positional coordinates of the images of the respective fluorescent objects on the membrane filter are obtained in advance, the images of the respective fluorescent objects can be accurately and automatically measured in an unmanned fashion by sequentially scanning upon changing the magnification of the objective lens from 10x, set in the above operation, to, for example, 20x or 40x (this operation will be referred to as Validation operation). This further facilitates determination of microbe and others.

Note that the measurement data and the images of the respective fluorescent objects which are obtained by the above method are filed and stored in the image analyzing unit 3. This file can be arbitrarily read out to be referred, as needed, under the control of the image analyzing unit 3.

### [Image Processing by Binarization Technique for Fluorescent Objects Step: S96 in Fig. 5]

Image processing (binarization technique) will be described next, which is to be performed for an image of a fluorescent object obtained in the entire region of the sample described above before the image analyzing unit 3 performs image analysis.

An image to be processed by the image analyzing unit 3 has multi-tone digital information. For example, monochrome images use 256 gray levels (8-bit gray levels).

The image analyzing unit 3 can have a function of digitizing the captured image. In the embodiment, since a digital camera is used as the image capturing unit 21, the captured image is already digitized and is a multi-tone digital image (256 gray levels (8-bit gray levels).

The image analyzing unit 3 then performs image processing by a binarization technique. In binarization, each pixel constituting this multi-tone image is "binarized" by setting a luminance within an arbitrary range as "active" and other luminances as "negative", thereby converting an 8-bit gray-level image into a 1-bit gray-level image.

### [Extraction of Concatenated component: Fig. 4]

A method of extracting a concatenated component necessary for identification processing of a microbe by using the above 1-bit gray-level binary image obtained for each measurement area on the membrane filter.

Fig. 4 shows an example of a binary image obtained by performing image processing by the above binarization method for the image obtained by measuring the measurement area 80 as a predetermined area.

Of the pixels 81 located on the central portion in Fig. 4, the "cluster" obtained by connecting the "active" pixels (hatched portion) having luminances equal to or higher than a predetermined luminance is defined as the concatenated component 82. The area occupied by the concatenated component 82 is the occupied area 83 of the concatenated component. The concatenated component end point 84 indicates the coordinates of the lowermost-rightmost pixel on the frame of the occupied area of the concatenated component.

Referring to Fig. 4, the area, average luminance, curve length, curve width, and roundness of the occupied area 83 can be calculated from the concatenated component 82 as the cluster of the "active" pixels having luminances equal to or higher than the predetermined luminance by using the image analysis method to be described later.

### [Secondary Automatic Identification Processing: 1-Wavelength Identification Method: Fig. 6]

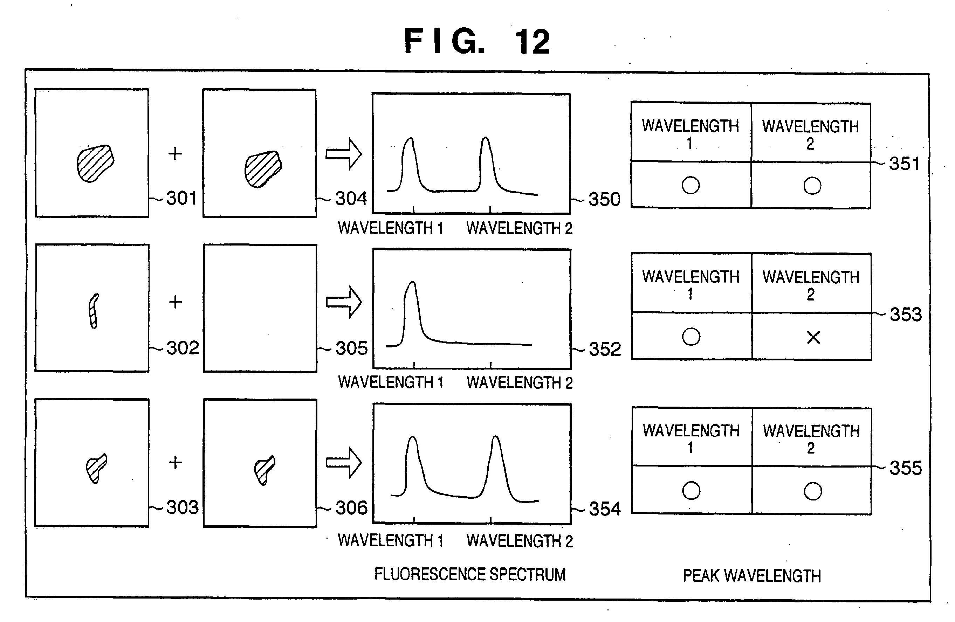

Three kinds of identifying methods, i.e., a 1-wavelength identifying method (Fig. 6), 2-wavelength identifying method (Fig. 7), and 3-wavelength identifying method (Fig. 12), will be described in detail next as secondary automatic identification processing of identifying microbes from fluorescent objects extracted by primary automatic identification processing.

The secondary automatic identification processing is performed by using a high-power lens to accurately identify microbes.

The secondary automatic identification processing based on the 1-wavelength identification method will be described first.

Fig. 6 is a flow chart for secondary automatic identification processing using one wavelength. This processing is executed by the image analyzing unit 3 on the basis of an automatic fluorescence inspection program.

Referring to Fig. 6, the flow advances from step S99 in Fig. 5 to step S195 to move the stage in accordance with the location map of fluorescent objects extracted by the primary automatic identification processing.

Upon completion of the processing in steps S95 and S96, the flow advances to step S196. Note that the processing in steps S95 and S96 in Fig. 6 is the same as that in the steps denoted by the same reference symbols as in Fig. 5. A repetitive description of this processing will be omitted.

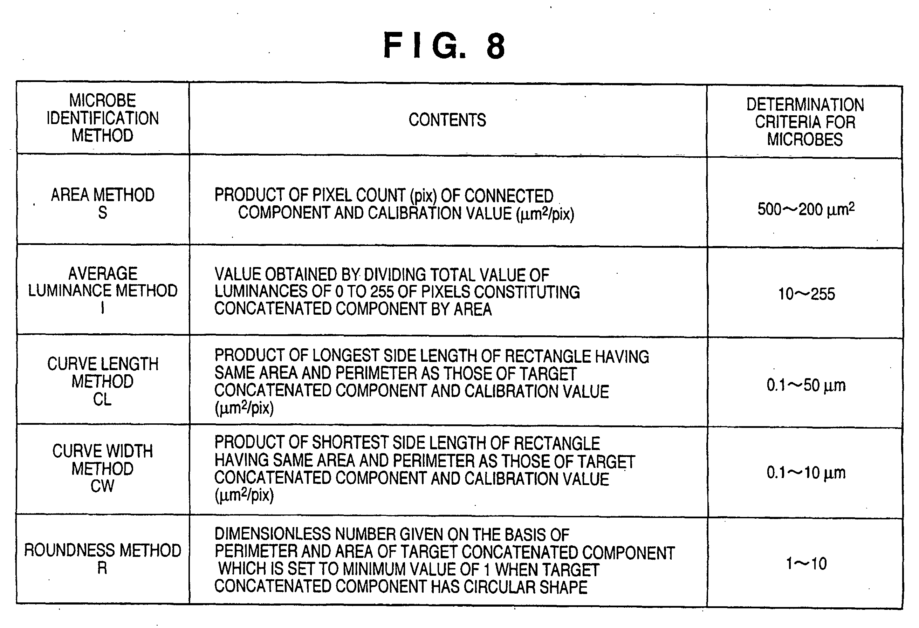

Step S196 is a step which characterizes automatic microbe identification processing using excitation light of one wavelength. As automatic microbe identification processing, for example, an area method, average luminance method, and curve length method are available, which specify fluorescent objects which can be microbes on the basis of the fluorescence intensity and shape of images of fluorescent objects. These methods will be described in detail below with reference to Figs. 8 to 10.

Fig. 8 shows the area method, average luminance method, curve length method, curve width method, and roundness method, each exemplifying a microbe identification processing method using excitation light of one wavelength, and determination criteria for microbes in the respective methods.

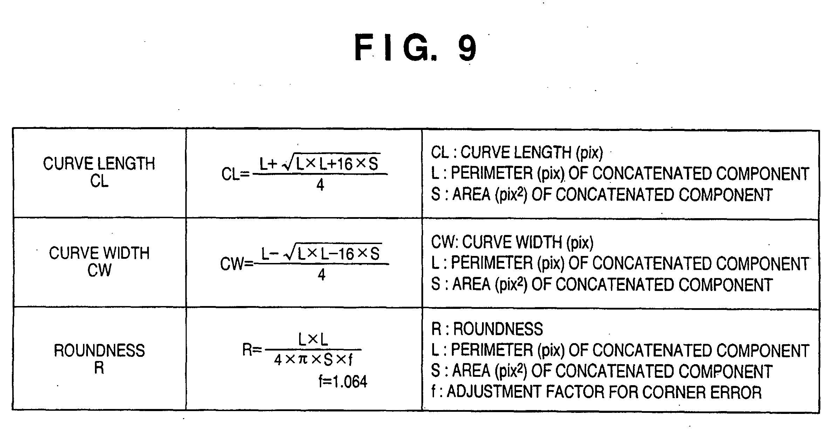

Fig. 9 shows equations for calculating a curve length, curve width, and roundness in the curve length method, curve width method, and roundness method shown in Fig. 8.

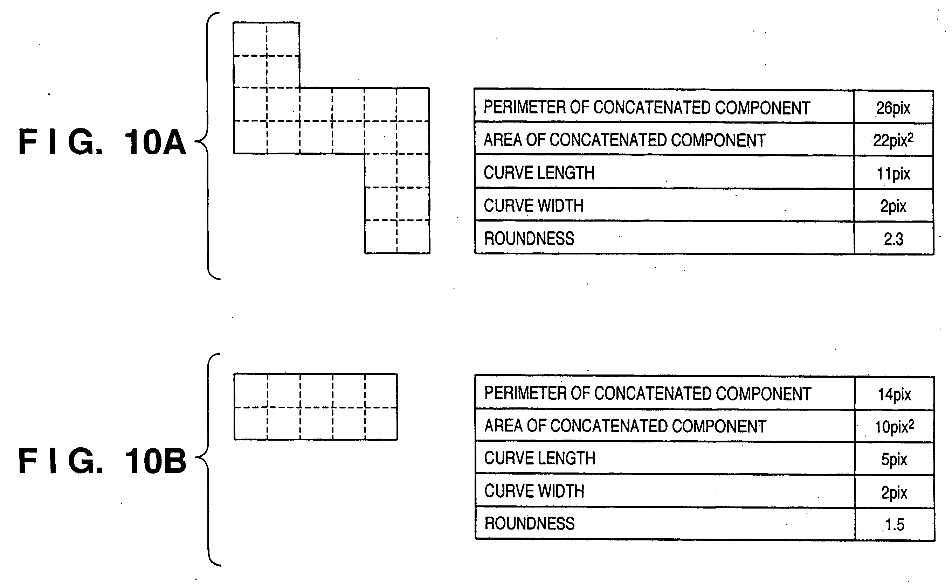

Fig. 10 shows an example of how curve lengths, curve widths, and roundness are calculated from specific fluorescent images by using the curve length method, curve width method, and roundness method.

### [Area Method: Fig. 8]

The area method will be described first.

As shown in Fig. 8, in the area method, the actual area ((µm)²) of a concatenated component obtained by image sensing is calculated, which is the product of the total number of pixels (pix) of the concatenated component and a calibration value ((µm)²/pix) which is formed in advance and an actual area per unit pixel. The obtained actual area is then compared with a preset determination criterion (Fig. 8) to discriminate whether or not the concatenated component is a microbe. For example, a determination criterion (Fig. 8) is set such that if the actual area of a concatenated component is 5 to 200 (µm)², the concatenated component is identified as a microbe.

### [Average Luminance Method: Fig. 8]

The average luminance method will be described next.

The average luminance method is a method of obtaining an average luminance from the luminance (0 to 255) of each pixel constituting a concatenated component, as shown in Fig. 8. An average luminance is obtained by dividing the total luminance of the respective pixels by the total number of pixels (pix). For example, a determination criterion (Fig. 8) is set such that if the average luminance of a concatenated component is 10 to 255, the concatenated component is identified as a microbe.

### [Curve Length Method: Fig. 8]

The curve length method will be described next.

As shown in Fig. 8, in the curve length (CL) method, a curve length (µm) is calculated, which is the product of the length (pix) of the longest pixel side of a rectangle having the same area and perimeter as those of a target concatenated component and a calibration value (µm/pix) which is formed in advance and a unit pixel length.

For example, the curve length of the target concatenated component in Fig. 10A is 11 (pix), and the curve length of the target concatenated component in Fig. 10B is 5 (pix).

The obtained curve length is then compared with a preset microbe determination criterion (Fig. 8) to discriminate whether or not the concatenated component is a microbe.

Note that the length of the longest pixel side of a rectangle having the same area and perimeter as those of a target concatenated component is calculated by the definition equation shown in Fig. 9. For example, a microbe determination criterion is set such that if the curve length is 0.5 to 50 µm, the concatenated component is identified as a microbe. Note that the value of a curve length indicates the length of a curved microbe, fiber, or the like.

### [Curve Width Method: Fig. 8]

The curve width method will be described next.

As shown in Fig. 8, in the curve width (CW) method, a curve length (µm) is calculated, which is the product of the length (pix) of the shortest side of a rectangle having the same area and perimeter as those of a target concatenated component and a calibration value (µm/pix) which is formed in advance and a unit pixel length.

For example, the curve width of the target concatenated component in Fig. 10A is 2 (pix), and the curve width of the target concatenated component in Fig. 10B is 2 (pix).

The obtained curve width is then compared with a preset microbe determination criterion (Fig. 8) to discriminate whether or not the concatenated component is a microbe.

Note that the length of the shortest pixel side of a rectangle having the same area and perimeter as those of a target concatenated component is calculated by the definition equation shown in Fig. 9. For example, a microbe determination criterion is set such that if the curve width is 0.1 to 10 µm, the concatenated component is identified as a microbe. Note that the value of a curve width indicates the width of a curved microbe, fiber, or the like.

### [Roundness Method: Fig. 8]

The roundness method will be described next.

In the roundness (R) method, a roundness is a dimensionless number given by the definition equation shown in Fig. 9, which is set to a minimum value of 1 when the target concatenated component has a circular shape, and is set to a value larger than 1 when the target concatenated component has a shape other than the circular shape.

For example, the roundness of the target concatenated component in Fig. 10A is 2.3, and the roundness of the target concatenated component in Fig. 10B is 1.5.

Note that in the definition equation shown in Fig. 9, 1.064 is an adjustment factor, which corrects corner errors caused by digitization of an image throughout the circumference. For example, a microbe determination criterion (Fig. 8) is set such that if the curve width is 1 to 10 µm, the concatenated component is identified as a microbe.

If it is determined in step S197 in Fig. 6 that there is a fluorescent object for which automatic identification processing is to be performed, the flow returns to step S195 to repeat the above processing from step S195 to step S196. If it is determined in step S197 that there is no fluorescent object for which automatic identification processing is to be performed, the flow advances to step S198.

In step S198, the location map of microbes and others is created on the basis of the determination obtained in step S196 with respect to each fluorescent object extracted in step S99, and the detected microbes are displayed on the display screen. On the display screen, microbes and others can be displayed by three kinds of discrimination methods.

In step S199, the microbe determination results on the respective fluorescent objects which are obtained by image analysis processing are stored, thereby completing automatic microbe identification processing using excitation light of one wavelength.

In this manner, the 1-wavelength identification method can identify microbes from the characteristic features of the shapes of fluorescent objects.

### [Secondary Automatic Identification Processing: 2-wavelength Identification Method: Fig. 7]

As the second method of secondary automatic identification processing of identifying microbes from fluorescent objects extracted by primary automatic identification processing, the 2-wavelength identification method using excitation light beams of two wavelengths will be described in detail next.

In the 2-wavelength identification method, a sample is stained in advance with a fluorescent dye in Fig. 3 to make a microbe to be detected, i.e., a target microbe, emit fluorescence when irradiated with specific excitation light.

An outline of the 2-wavelength identification method will be described first. A target microbe contained in a sample is stained with one kind of fluorescent dye. The sample is sequentially irradiated with excitation light corresponding to the fluorescent dye and other excitation light other than this. Fluorescent images emitted from each fluorescent object are sequentially detected, and a fluorescence spectrum is created for each fluorescent object by combining the fluorescent images obtained in correspondence with the respective excitation light beams. The obtained fluorescence spectrum is compared with a preset fluorescence spectrum as a determination criterion. Each fluorescent object is then identified as a target microbe or a object other than the target microbe. This makes it possible to identify target microbes in the sample.

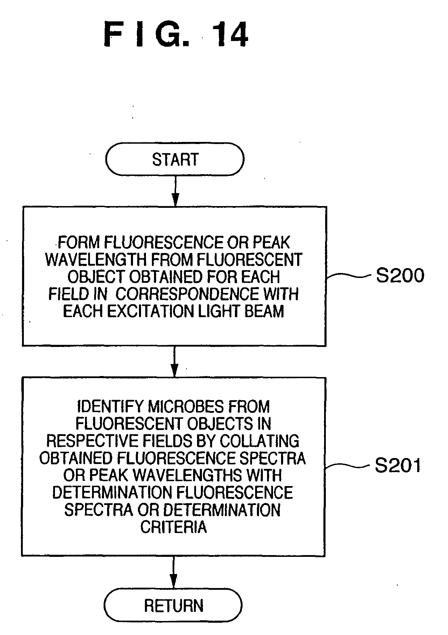

Figs. 7 and 14 are flow charts for automatic microbe identification processing using two wavelengths. This processing is executed by the image analyzing unit 3 on the basis of the automatic fluorescence inspection program.

Referring to Fig. 7, the flow advances from step S99 in Fig. 5 to step S293 to move the stage in accordance with the location map of fluorescent objects extracted by primary automatic identification processing.

The processing in steps S95, S96, and S196 is performed by using excitation light having the first wavelength to specify fluorescent objects that can be target microbes from characteristic features such as the shapes of the fluorescent objects according to the microbe determination criterion shown in Fig. 8. The flow then advances to step S294. Note that the processing in steps S95, S96, and S196 in Fig. 7 is the same as that in the steps denoted by the same reference symbols in Fig. 6, and hence a detailed repetitive description will be omitted.

In step S294, the fluorescence filter is switched to another filter. The flow then advances to step S295 to repeatedly perform the above processing in steps S95, S96, and S196 by using excitation light having the second wavelength.

When the processing in step S295 is complete, the flow advances to step S296. In step S296, target microbes are identified among the fluorescent objects specified in step S196 which can be the respective target microbes. Step S296 is a step which characterizes automatic microbe identification processing using excitation light beams of two wavelengths. Fig. 14 shows this step in detail. In step S200, a fluorescence spectrum or peak wavelength is created from a fluorescent object obtained for each field in correspondence with each excitation light beam. In step S201, the obtained fluorescence spectrum or peak wavelength is collated with a determination fluorescence spectrum or determination criterion to identify a microbe from the fluorescent object in each field.

If it is determined in step S297 that there is a fluorescent object for which automatic identification processing is to be performed next, the flow advances to step S298 to return the fluorescence filter to the original position. The flow then returns to step S293 to repeatedly perform the above processing from step S293 to step S296. If it is determined in step S297 that there is no fluorescent object for which automatic identification processing is to be performed next, the flow advances to step S198 to perform the processing in steps S198 and S199.

Note that the processing in steps S198 and S199 in Fig. 7 is the same as that in the steps denoted by the same reference symbols in Fig. 6, and hence a detailed repetitive description will be omitted.

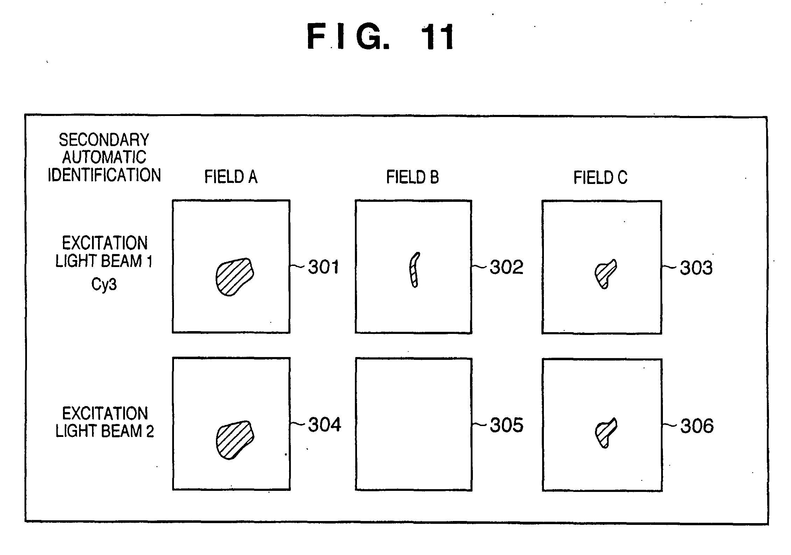

Secondary automatic identification processing in the above 2-wavelength identification method will be described in detail next with reference to Figs. 11 to 13. This processing is executed by the image analyzing unit 3 on the basis of the automatic fluorescence inspection program.

In secondary automatic identification processing using excitation light beams of two wavelengths, first of all, the area method, average luminance method, curve length method, or the like described in the 1-wavelength identification method is applied to each of excitation light beams of two wavelengths to specify fluorescent objects that can be target microbes, according to the microbe determination criterion shown in Fig. 8, from the characteristic features, e.g., the fluorescence intensities or shapes, of the fluorescent objects with respect to the respective excitation light beams (step S196). Microbes are then identified by using the differences between the fluorescent objects that can be the target microbes which are obtained with respect to the respective excitation light beams (step S296).

Fig. 11 is a diagram for explaining an example of fluorescent objects (binary images) in the respective fields which are obtained by secondary identification processing using excitation light beams of two wavelengths.

In secondary identification processing using excitation light beams of two wavelengths, a sample on a membrane filter is irradiated with two different excitation light beams 1 (for example: for detection of Cy3) and 2 to image-sense fluorescent objects obtained for the respective fields (three fields A, B, and C in this case) in correspondence with the respective excitation light beams, thereby acquiring fluorescent objects (binary images) 301 to 306, as shown in, for example, Fig. 11.

As shown in Fig. 12, the six fluorescent objects 301 to 306 obtained in the fields A, B, and C in correspondence with the two excitation light beams 1 and 2 are combined to form fluorescence spectra 350, 352, and 354 or peak wavelengths 351, 353, and 355.

For example, in the field A, the fluorescent objects 301 and 304 obtained in correspondence with the two excitation light beams are combined to form the fluorescence spectrum having two peaks or the peak wavelength 351. In the field B, the fluorescent objects 302 and 305 obtained in correspondence with the two excitation light beams are combined to form the fluorescence spectrum having one peak and or the peak wavelength 353. In the field C, the fluorescence spectrum 354 or peak wavelength 355 is formed in the same manner.

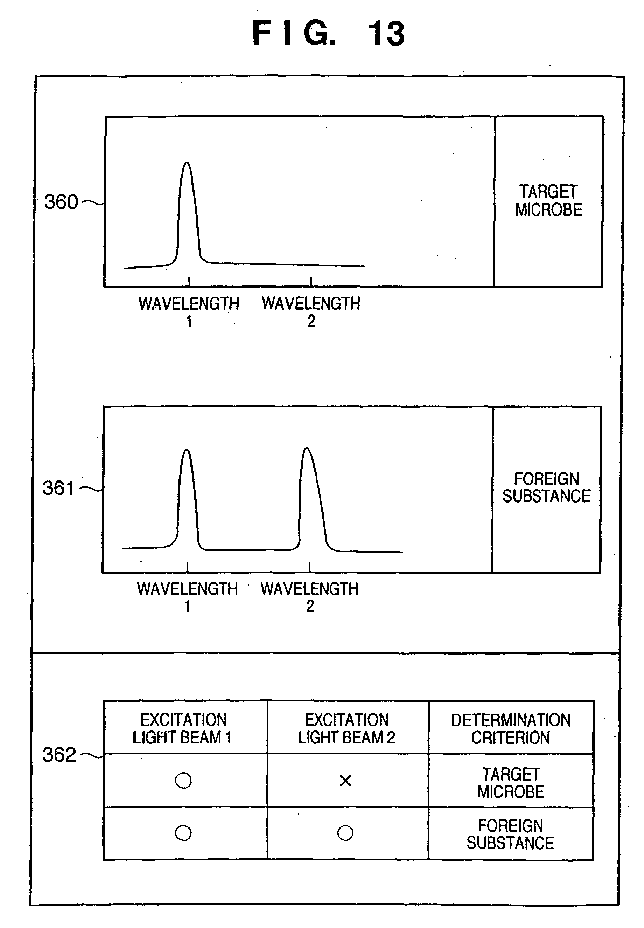

The formed fluorescence spectra 350, 352, and 354 or peak wavelengths 351, 353, and 355 are then compared with a determination fluorescence spectrum 360 indicating a target microbe, a determination fluorescence spectrum 361 indicating a foreign object, or a determination criterion (for two wavelengths) 362, each of which is shown in Fig. 13 as an example. A determination fluorescence spectrum or determination criterion is set to determine from the distribution of fluorescence peaks obtained in advance in correspondence with each excitation light beam whether the fluorescence spectrum or peak wavelength corresponds to the target microbe or foreign object. For example, by comparing the fluorescence spectra 350, 352, and 354 obtained in the respective fields in Fig. 11 with the determination fluorescence spectrum 360 indicating the target microbe and the determination fluorescence spectrum 361 indicating the foreign object, only the fluorescent object in the field B of the fluorescent objects obtained in the three fields in Fig. 11 is identified as the target microbe, and the fluorescent objects in the fields A and C are identified as foreign objects. Note that an autofluorescent object with no fluorescence selectivity with respect to excitation light might be an example of foreign object. The determination fluorescence spectra or determination criterions used in the above operation are stored in advance in the image analyzing unit 3 in correspondence with the respective excitation light beams.

In this manner, fluorescence spectra or peak wavelengths are formed from the fluorescent objects (binary images) 301 to 306 and are compared with a determination fluorescence spectrum or determination criterion. This makes it possible to automatically identify each fluorescent object as the target microbe or a foreign object. Therefore, a target microbe and the like contained in a sample solution can be easily identified in an unmanned fashion.

### [Secondary Automatic Identification Processing: "3 or More" Wavelength Identification Method: Fig. 15]

As the third method of secondary automatic identification processing of identifying microbes from fluorescent objects extracted by primary automatic identification processing, a "3 or more" wavelength identification method using excitation light beams of three or more wavelengths will be described in detail next by taking the 3-wavelength identification method using excitation light beams of three wavelengths as an example. This processing is executed by the image analyzing unit 3 on the basis of the automatic fluorescence inspection program.

In the identification method using three wavelengths, a sample is stained with two kinds of fluorescent dyes (e.g., Cy3 and DAPI) to identify a target microbe and microbes other than the target microbe contained in the sample. For example, a target microbe (e.g., Pectinatus) is dually stained with two kinds of fluorescent dyes (Cy3 and DAPI), and microbes other than the target are stained with only one kind of fluorescent dye (DAPI).

The 3-wavelength identification method will be described first. The microbes contained in a sample are stained with two kinds of fluorescent dyes such that a target microbe and other microbes can be identified. The sample is sequentially irradiated with two kinds of excitation light beams corresponding to the fluorescent dyes and other kinds of excitation light beams to sequentially detect fluorescent images emitted from the respective fluorescent objects. The fluorescent images obtained in correspondence with the respective excitation light beams are combined to form fluorescence spectra for the respective fluorescent objects. The obtained fluorescence spectra are compared with a determination fluorescence spectrum. This makes it possible to identify each fluorescent object as the target microbe or another microbe or another object, thus identifying the target microbe in the sample.

Figs. 15 and 14 are flow charts for automatic microbe identification processing using three wavelengths.

Referring to Fig. 15, the flow advances from step S99 in Fig. 5 to step S293 to move the stage in accordance with the location map of fluorescent objects extracted by the primary automatic identification processing.

The processing in steps S95, S96, and S196 is performed by using excitation light having the first wavelength to specify fluorescent objects that can be target microbes from characteristic features such as the fluorescence intensities or shapes of the fluorescent objects according to the microbe determination criterion shown in Fig. 8. The flow then advances to step S294. Note that the processing in steps S95, S96, and S196 in Fig. 15 is the same as that in the steps denoted by the same reference symbols in Fig. 6, and hence a detailed repetitive description will be omitted.

The flow then advances to step S390. If it is determined that the current fluorescence filter needs to be switched to the next fluorescence filter for irradiation with next excitation light, the flow advances to step S294 to switch the fluorescence filters. The flow then advances to step S295 to repeatedly perform the above processing in steps S95 and S96 by using excitation light having the second wavelength. Thereafter, the flow advances to step S396. If it is determined in step S390 that irradiation of the sample with all excitation light beams is complete, and there is no need to switch to the next filter, the flow advances to step S396.

In step S396, a target microbe is identified among the fluorescent objects specified in step S196 which can be the respective target microbes. Step S396 is a step which characterizes automatic microbe identification processing using excitation light beams of three or more wavelengths. Fig. 14 shows this step in detail. In step S200, a fluorescence spectrum or peak wavelength is created from a fluorescent object obtained for each field in correspondence with each excitation light beam. In step S201, the obtained fluorescence spectrum or peak wavelength is collated with a determination fluorescence spectrum or determination criterion to identify a microbe from the fluorescent object in each field.

If it is determined in step S297 that there is a fluorescent object for which automatic identification processing is to be performed next, the flow advances to step S298 to return the fluorescence filter to the original position. The flow then returns to step S293 to repeatedly perform the above processing from step S293 to step S396. If it is determined in step S297 that there is no fluorescent object for which automatic identification processing is to be performed next, the flow advances to step S198 to perform the processing in steps S198 and S199.

Note that the processing in steps S198 and S199 in Fig. 12 is the same as that in the steps denoted by the same reference symbols in Fig. 6, and hence a detailed repetitive description will be omitted.

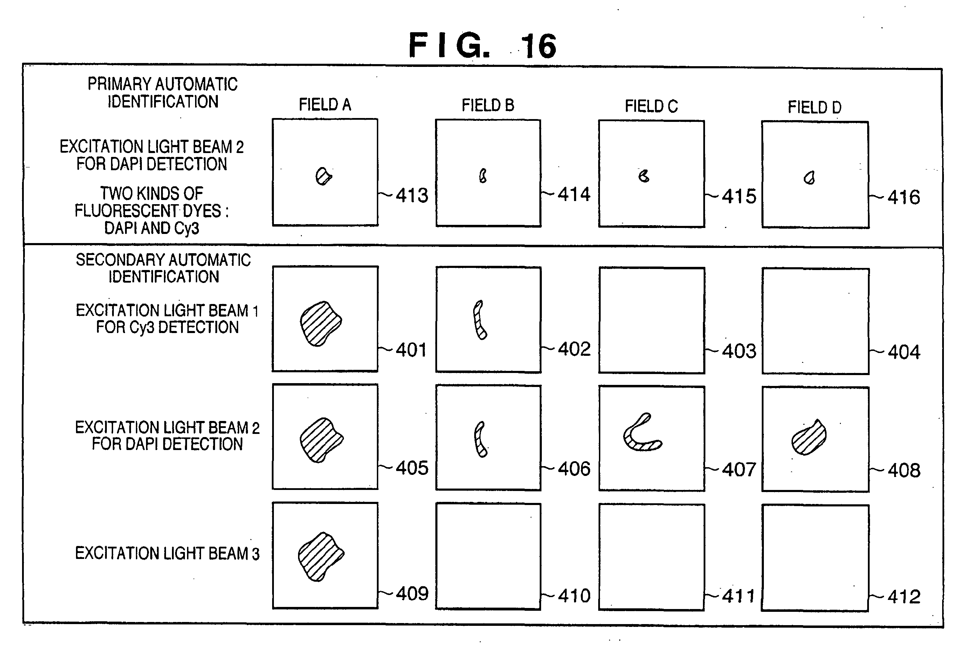

Secondary automatic identification processing in the above 3-wavelength identification method will be described in detail next with reference to Figs. 16 to 20.

In secondary automatic identification processing using excitation light beams of three wavelengths, first of all, the area method, average luminance method, curve length method, or the like described in the 1-wavelength identification method is applied to each of excitation light beams of three wavelengths to specify fluorescent objects that can be target microbes from the fluorescence intensities or shapes of the fluorescent objects with respect to the respective excitation light beams (step S196). Microbes are then identified by using the differences between the fluorescent objects that can be the target microbes which are obtained with respect to the respective excitation light beams (step S396).

Fig. 16 is a diagram for explaining an example of fluorescent objects (binary images) in the respective fields which are obtained by primary automatic identification processing and secondary identification processing using excitation light beams of three wavelengths.

In primary automatic identification processing using excitation light beams of three wavelengths, a low-power lens is used to irradiate a sample on a membrane filter with, for example, excitation light beam 2 (for DAPI detection) and image-sense fluorescent objects obtained for the respective fields in correspondence with excitation light beam 2, thereby capturing fluorescent objects (binary images) 413 to 416.

In secondary automatic identification processing using excitation light beams of three wavelengths, only the fields in which fluorescent objects were detected by the primary automatic identification processing are sequentially irradiated with three different excitation light beams 1 to 3. As shown in, for example, Fig. 16, the fluorescent objects obtained in the respective fields (four fields A to D in this case) in correspondence with the respective excitation light beams are then image-sensed to acquire fluorescent objects (binary images) 401 to 412.

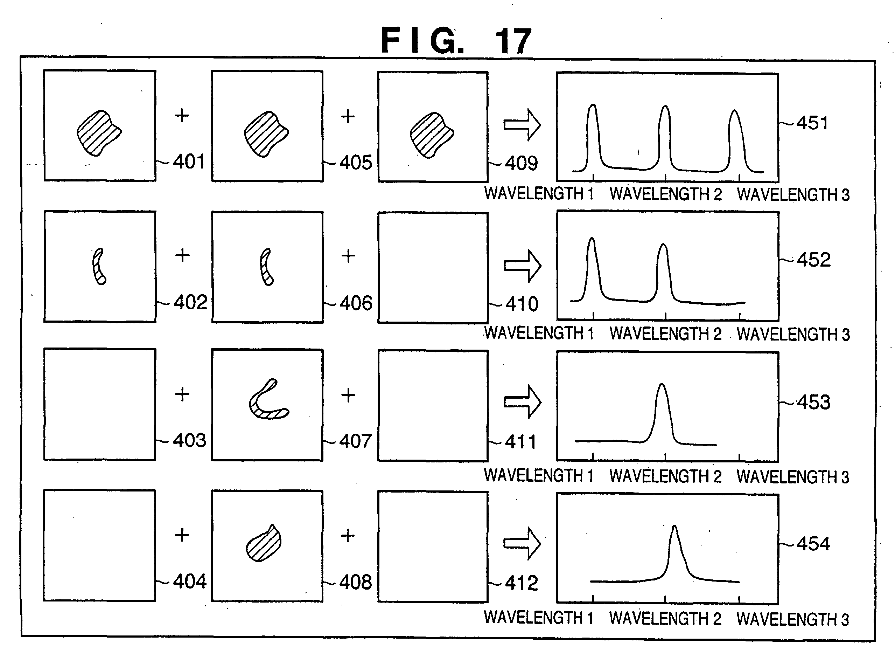

As shown in Fig. 17, three each of the fluorescent objects 401 to 412 obtained in the fields A to D in correspondence with three excitation light beams 1 to 3 are combined to form fluorescence spectra 451 to 454. Although not shown in Fig. 17, peak wavelengths like those shown in Fig. 12 may be formed in place of the fluorescence spectra 451 to 454.

For example, in the field A, the fluorescent objects 401, 405, and 409 obtained in correspondence with the three excitation light beams are combined to form the fluorescence spectrum 451 having three peaks. In the field B, the fluorescent objects 402, 406, and 410 obtained in correspondence with the three excitation light beams are combined to form the fluorescence spectrum 452 having two peaks. In the fields C and D, the fluorescence spectra 453 and 454 each having one peak are formed in the same manner.

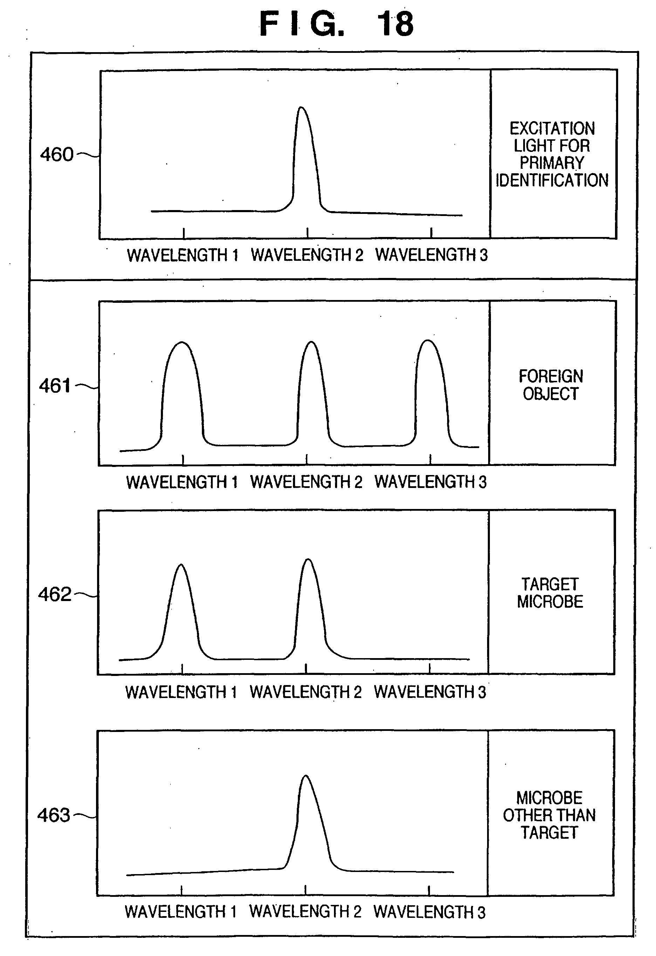

The formed fluorescence spectra 451 to 454 are then compared with determination fluorescence spectra 460 to 463 exemplarily shown in Fig. 18. The determination fluorescence spectra are prepared in correspondence with the excitation light used for primary identification and the combinations of excitation light beams used for secondary identification to determine from the obtained distributions of fluorescence peaks whether the corresponding fluorescence spectra correspond to target microbes, microbes other than target microbes, or foreign objects.

Note that the determination fluorescence spectra 461 to 463 exemplarily shown in Fig. 18 are examples of determination fluorescence spectra for 3-wavelength identification processing, which are used for secondary identification using excitation light beams 1 to 3 with respect to the fluorescent objects detected from the sample upon irradiation with excitation light beam 2 as a primary identification excitation light beams denoted by reference numeral 460. The spectra 461, 462, and 463 respectively indicate a foreign object, a target microbe, and a microbe other than the target microbe.

Collating the fluorescence spectra 451 to 454 obtained for the respective fields with the determination fluorescence spectra 461 to 463 will identify only the fluorescent object in the field B, of the fluorescent objects obtained in the four fields in Fig. 16, as the target microbe, the fluorescent objects in the fields C and D as microbes other than the target, and the fluorescent object in the field A as a foreign object. Note that an autofluorescent object with no fluorescence selectivity with respect to excitation light may be an example of foreign object. The determination fluorescence spectra used in the above operation are stored in advance in the image analyzing unit 3 in correspondence with the respective excitation light beams.

The peak wavelengths shown in Fig. 12 may be formed in place of the above fluorescence spectra. In this case, determination criteria for 3-wavelength identification processing like those shown in Fig. 13, which are stored in the image analyzing unit 3, may be used in place of the determination fluorescence spectra.

Forming fluorescence spectra or peak wavelengths from the fluorescent objects (binary images) 401 to 412 and comparing them with determination fluorescence spectra or determination criteria in this manner makes it possible to automatically identify the fluorescent objects as target microbes, microbes other than target microbes, or foreign objects. Therefore, a target microbe and the like contained in a sample solution can be easily identified in an unmanned fashion.

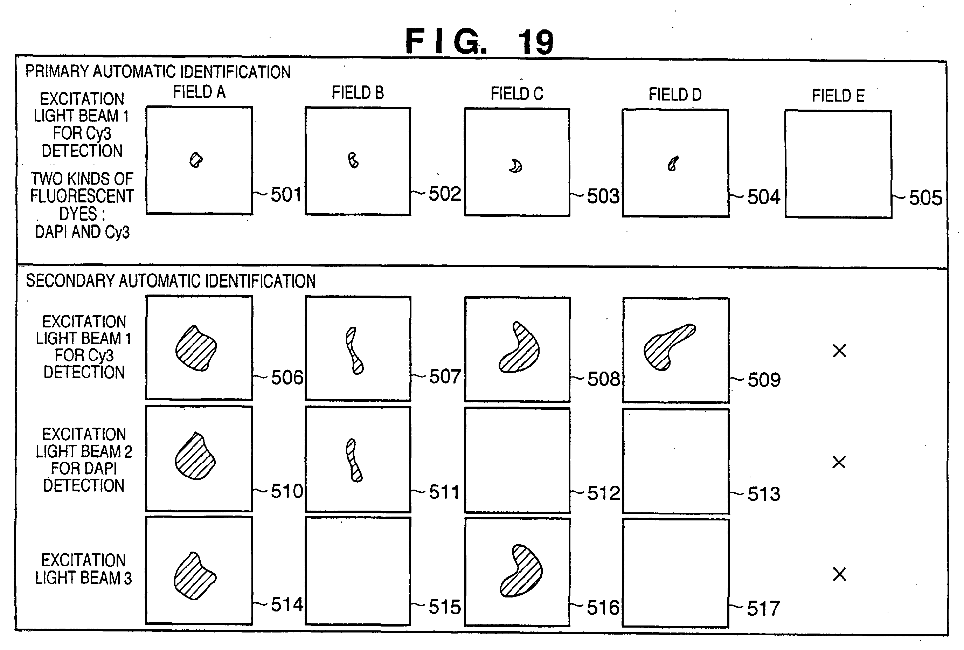

### [Another Example of 3-Wavelength Identification Method: Fig. 19]

Fig. 19 is a diagram for explaining another example of secondary automatic identification processing using excitation light beams of three wavelengths. A sample is stained in advance with two kinds of fluorescent dyes (e.g., Cy3 and DAPI) to identify a target microbe and microbes other than the target microbe contained in the sample.

The example shown in Fig. 19 differs from that shown in Fig. 16 in the following point. In Fig. 16, excitation light beams 2 (for DAPI detection) is used as an excitation light beam used in primary identification processing of detecting fluorescent objects at a low magnification before secondary identification processing. In contrast to this, in Fig. 19, excitation light beam 1 (for Cy3 detection) is used as an excitation light beam used in primary identification processing.

Reference numerals 501 to 505 in Fig. 19 denote fluorescent objects detected in primary identification processing. They are binary images of fluorescent objects detected from the respective fields (five fields A to E in this case) in correspondence with excitation light beam 1 upon irradiation of a sample on a membrane filter with excitation light beam 1 (for Cy3 detection). Note that no fluorescent object is obtained from the field E.

As shown in Fig. 19, three each of fluorescent objects 506 to 517 obtained in the respective fields A to D in correspondence with three excitation light beams 1 to 3 are combined to form fluorescence spectra like those shown in Fig. 17 or peak wavelengths like those shown in Fig. 12, although they are not shown.

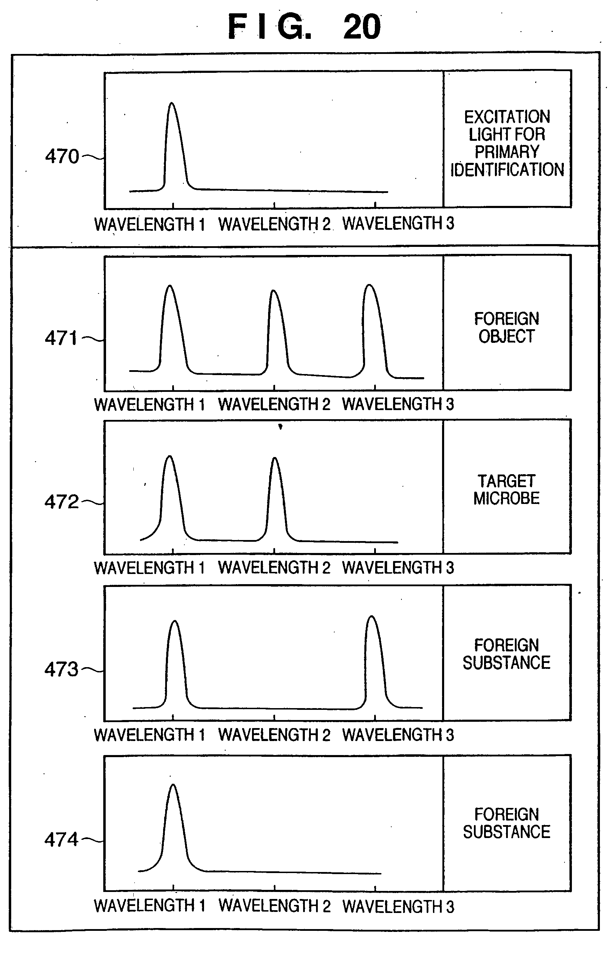

The formed fluorescence spectra are then compared with determination fluorescence spectra 471 to 474 for 3-wavelength identification processing, respectively, which are exemplarily shown in Fig. 20. Note that the determination fluorescence spectra shown in Fig. 20 are prepared in correspondence with excitation light which is denoted by reference numeral 470 and used for primary identification and the combinations of excitation light beams used for secondary identification to determine from the obtained distributions of fluorescence peaks whether the corresponding fluorescence spectra correspond to target microbes, microbes other than target microbes, or foreign objects.

Note that the determination fluorescence spectra 471 to 474 exemplarily shown in Fig. 20 are examples of determination fluorescence spectra for 3-wavelength identification processing, which are used for secondary identification using excitation light beams 1 to 3 with respect to the fluorescent objects detected from the sample upon irradiation with excitation light beam 1 as a primary identification excitation light beams denoted by reference numeral 470. The spectrum 471 indicates a foreign object; the spectrum 472, a target microbe; and the spectra 473 and 474, foreign objects.

Collating the fluorescence spectra (not shown) obtained for the respective fields with the determination fluorescence spectra 471 to 474 will identify only the fluorescent object in the field B, of the fluorescent objects obtained in the four fields in Fig. 19, as the target microbe, and the fluorescent objects in the fields A, C and D as foreign objects. Note that an autofluorescent object with no fluorescence selectivity with respect to excitation light is an example of foreign object. The determination fluorescence spectra used in the above operation are stored in advance in the image analyzing unit 3 in correspondence with the respective excitation light beams.

Forming fluorescence spectra or peak wavelengths from the fluorescent objects (binary images) 506 to 517 and comparing them with determination fluorescence spectra or determination criteria in this manner makes it possible to automatically identify the fluorescent objects as target microbes or foreign objects. Therefore, a target microbe and the like contained in a sample solution can be easily identified in an unmanned fashion.

Referring to Fig. 19, since only excitation light beam 1 (for Cy3 detection) for identifying only the target microbe is used as an excitation light beam used in primary identification processing, microbes other than the target microbe are excluded by the primary automatic identification processing. For this reason, only the target microbe can be identified among the microbes contained in the sample by primary automatic identification processing. In addition, the identification method shown in Fig. 19 can accurately discriminate the target microbe (field B) from the foreign objects (fields A, C and D) among the fluorescent objects (fields A to D) identified by the primary identification processing.

As described above, the microbe inspection equipment of this embodiment can detect a trace amount of fluorescence, and hence can detect only one cell of target microbes in a sample. For this reason, unlike in the prior art, there is no need to take a long period of time to form a colony of microbes by cultivation to prepare a sample containing a large number of microbes.

In addition, various kinds of parameters such as the feature amounts of shapes, e.g., the average fluorescence intensities, areas, and the ratios of curve lengths to curve widths of concatenated components, are calculated from images of detected fluorescent objects, and it can be detected in an unmanned fashion on the basis of the calculated various parameters whether or not the images of the fluorescent objects originate from microbes. This eliminates the necessity to visually identify and check microbes as in the prior art, and hence allows accurate, quick, automatic detection of microbes.

A measurement equipment according to the present invention can therefore be applied to microbial tests in waste water, industrial water, environmental samples, and water and sewerage, microbial test in various research fields such as life-science, detection of minute autofluorescent objects and analysis of the number thereof, and the like as well as microbial tests in manufacturing process control, product quality control, and the like for beverages, foods, medicines, cosmetics, and the like.

As described above, according to the present invention, a microbe inspection equipment and method can be provided, which can automatically and quickly acquire information about microbes contained in a sample as a test target.

## Claims

1. A testing method of testing a microbe contained in a sample, **characterized by** comprising:
an irradiation step of irradiating the sample with a plurality of excitation light beams having different wavelengths; and
an identification step of identifying a microbe contained in the sample on the basis of a distribution of peaks of fluorescence obtained from each object contained in the sample in correspondence with irradiation with the plurality of excitation light beams.

2. The testing method according to claim 1, **characterized in that** the method further comprises an inspection step of specifying a fluorescent object that can be a microbe on the basis of shapes of fluorescent objects obtained from the respective objects, and in the identification step, a distribution of peaks of the fluorescence is obtained by using the fluorescent object specified in the inspection step.

3. The testing method according to claim 1, **characterized in that** the method further comprises an inspection step of specifying a fluorescent object that can be a microbe on the basis of fluorescence intensities of fluorescent objects obtained from the respective objects, and in the identification step, a distribution of peaks of the fluorescence is obtained by using the fluorescent object specified in the inspection step.

4. The testing method according to claim 1, **characterized in that** in the irradiation step, the sample is sequentially irradiated with the plurality of excitation light beams having different wavelengths.

5. The testing method according to claim 1, **characterized in that** in the irradiation step, the sample is simultaneously irradiated with the plurality of excitation light beams having different wavelengths.

6. The testing method according to claim 1, **characterized in that**
fluorescence obtained from each object contained in the sample has not less than one peak, and
in the identification step, a microbe contained in the sample is identified on the basis of each peak wavelength or frequency of fluorescence obtained from each object contained in the sample.

7. The testing method according to claim 6, **characterized in that** in the identification step, each peak wavelength or frequency of fluorescence obtained from each of the objects is collated with determination criteria defined in advance in correspondence with the plurality of excitation light beams to determine whether or not each of the objects is a microbe.

8. The testing method according to claim 7, **characterized in that** the microbe is a specific microbe.

9. The testing method according to claim 1, **characterized in that**
fluorescence obtained from each object contained in the sample has not less than one peak, and
in the identification step, a microbe contained in the sample is identified on the basis of a fluorescence spectrum obtained from each object contained in the sample.

10. The testing method according to claim 9, **characterized in that** in the identification step, a fluorescence spectrum obtained from each of the object is collated with determination fluorescence spectra defined in advance in correspondence with the plurality of excitation light beams to determine whether or not each of the objects is a microbe.

11. The testing method according to claim 10, **characterized in that** the microbe is a specific microbe.

12. The testing method according to claim 1, **characterized by** comprising
a primary inspection step, and
a secondary inspection step,
wherein in the primary inspection step, a fluorescent object contained in the sample is specified by observing an entire region of the sample at a first magnification while irradiating the sample with the excitation light, and
in the secondary inspection step, the irradiation step and the identification step are executed, and a distribution of peaks of fluorescence from each of the fluorescent objects is obtained while each fluorescent object specified in the primary inspection step is observed at a second magnification higher than the first magnification.

13. The testing method according to claim 1, **characterized by** comprising
a primary inspection step, and
a secondary inspection step,
wherein in the primary inspection step, a target microbe is separated from microbes other than the target microbe among fluorescent objects contained in the sample by observing an entire region of the sample at a first magnification while irradiating the sample with the excitation light, and
in the secondary inspection step, the irradiation step and the identification step are executed, and a distribution of peaks of fluorescence from each of the target microbes is obtained while each target microbe extracted in the primary inspection step is observed at a second magnification higher than the first magnification.

14. The testing method according to claim 1, **characterized by** further comprising a sample preparation step of capturing a microbe contained in the sample on a filter, and staining objects including the microbe captured on the filter with a fluorescent dye.

15. The testing method according to claim 1, **characterized by** further comprising a sample preparation step of capturing a microbe contained in the sample on a filter, and staining the microbe captured on the filter with a fluorescent dye such that the microbe captured on the filter has a peak in not less than one fluorescence upon irradiation of excitation light including not less than two wavelengths.

16. The testing method according to claim 15, **characterized in that** as the excitation light including not less than two wavelengths, not less than two excitation light beams selected from excitation light beams having wavelengths falling within a range of 340 nm to 750 nm at maximum intensities are used.

17. The testing method according to claim 15, **characterized in that** as the fluorescent dye, not less than one fluorescent dye selected from the group consisting of Texas Red, tetramethylrhodamine, indo-carbocyanine dye, Alexa dye, 4',6-diamidino-2-phenylindole (DAPI), providium iodide, and fluorescein isothiocyanate (FITC) is used.

18. The testing method according to claim 1, **characterized by** further comprising a sample preparation step of capturing a microbe contained in the sample on a filter, and staining the microbe captured on the filter with different kinds of fluorescent dyes such that the microbe captured on the filter has a peak in not less than two fluorescences upon irradiation of excitation light including not less than three wavelengths.

19. The testing method according to claim 18, **characterized in that** as the excitation light including not less than three wavelengths, not less than three excitation light beams selected from excitation light beams having wavelengths falling within a range of 340 nm to 750 nm at maximum intensities are used.

20. The testing method according to claim 18, **characterized in that** as the fluorescent dye, not less than two fluorescent dyes selected from the group consisting of Texas Red, tetramethylrhodamine, indo-carbocyanine dye, Alexa dye, 4',6-diamidino-2-phenylindole (DAPI), providium iodide, and fluorescein isothiocyanate (FITC) are used.

21. An inspection equipment for testing a microbe contained in a sample, **characterized by** comprising:
an irradiation mechanism which irradiates the sample with a plurality of excitation light beams having different wavelengths;
an image sensing device which image-senses the sample; and
an analyzing device which analyzes an image sensing result obtained by said image sensing device,
wherein said analyzing device is configured to identify, on the basis of the image sensing result, a microbe contained in the sample on the basis of a distribution of peaks of fluorescence obtained from each object contained in the sample in correspondence with irradiation with the plurality of excitation light beams.

22. The inspection equipment according to claim 21, **characterized in that** said analyzing device is configured to specify first a fluorescent object that can be a microbe on the basis of shapes of fluorescent objects obtained from the respective objects and then obtain a distribution of peaks of the fluorescence is obtained by using the fluorescent object specified.

23. The inspection equipment according to claim 21, **characterized in that** said analyzing device is configured to specify first a fluorescent object that can be a microbe on the basis of fluorescence intensities of fluorescent objects obtained from the respective objects and then obtain a distribution of peaks of the fluorescence is obtained by using the fluorescent object specified.

24. An inspection equipment for testing a microbe contained in a sample, **characterized by** comprising:
an input device which receives a result obtained by image-sensing the sample while irradiating the sample with a plurality of excitation light beams having different wavelengths; and
an analyzing device which identifies a microbe contained in the sample on the basis of a distribution of peaks of fluorescence obtained from each object contained in the sample in correspondence with the plurality of excitation light beams in accordance with the image sensing result received by said input device.

25. The inspection equipment according to claim 21, **characterized in that** said irradiation mechanism sequentially irradiates the sample with the plurality of excitation light beams having different wavelengths.

26. The inspection equipment according to claim 21, **characterized in that** said irradiation mechanism simultaneously irradiates the sample with the plurality of excitation light beams having different wavelengths.

27. The inspection equipment according to claim 21, **characterized in that**
fluorescence obtained from each object contained in the sample has not less than one peak, and
said analyzing device identifies a microbe contained in the sample on the basis of each peak wavelength or frequency of fluorescence obtained from each object contained in the sample.

28. The inspection equipment according to claim 27, **characterized in that** said analyzing device collates each peak wavelength or frequency of fluorescence obtained from each of the objects with determination criteria defined in advance in correspondence with the plurality of excitation light beams to determine whether or not each of the objects is a microbe.

29. The inspection equipment according to claim 21, **characterized in that** fluorescence obtained from each object contained in the sample has not less than one peak, and
said analyzing device identifies a microbe contained in the sample on the basis of a fluorescence spectrum obtained from each object contained in the sample.

30. The inspection equipment according to claim 29, **characterized in that** said analyzing device collates a fluorescence spectrum obtained from each of the object with determination fluorescence spectra defined in advance in correspondence with the plurality of excitation light beams to determine whether or not each of the objects is a microbe.

31. The inspection equipment according to claim 21, **characterized by** further comprising a control device which controls said irradiation mechanism and said image sensing device, said control device performing control to image-sense an entire region of the sample by using said image sensing device at a first magnification while irradiating the sample with the excitation light by using said irradiation mechanism and specify a fluorescent object contained in the sample by analyzing an image sensing result obtained by said image sensing device by using said analyzing device, and
then performing control to image-sense only each of the specified fluorescent objects by using said image sensing device at a second magnification higher than the first magnification while irradiating each of the specified fluorescent objects with the excitation light by using said irradiation mechanism and obtain a distribution of peaks of fluorescence from each of the fluorescent objects by analyzing an image sensing result obtained by said image sensing device by using said analyzing device.

32. The inspection equipment according to claim 21, **characterized by** further comprising a control device which controls said irradiation mechanism, said image sensing device, and said analyzing device,
said control device performing control to image-sense an entire region of the sample by using said image sensing device at a first magnification while irradiating the sample with the excitation light by using said irradiation mechanism and extract a target microbe, among fluorescent objects contained in the sample, while separating the target microbe from a microbe other than the target microbe by analyzing an image sensing result obtained by said image sensing device by using said analyzing device, and
performing control to image-sense each of the extracted target microbes by using said image sensing device at a second magnification higher than the first magnification and obtain a distribution of peaks of fluorescence from the target microbe by analyzing an image sensing result obtained by said image sensing device by using said analyzing device.

33. The inspection equipment according to claim 21, **characterized in that** as the plurality of excitation light beams, not less than two excitation light beams selected from excitation light beams having wavelengths falling within a range of 340 nm to 750 nm at maximum intensities are used.

34. The inspection equipment according to claim 32, **characterized in that**
said image sensing device further comprises a motor-driven stage, and
said control device controls said image sensing device to image-sense an entire region of the sample while controlling said motor-driven stage to scan the entire region of the sample.

35. The inspection equipment according to claim 34, **characterized in that** said image sensing device comprises an objective lens and an equation which is set in advance to keep a distance between the objective lens and a surface of the filter constant, and said control device controls said image sensing device on the basis of the equation to image-sense the entire region of the sample while keeping the distance between the objective lens and the surface of the filter constant so as not to cause an out-of-focus state in the scanning.

36. A control program which controls an inspection equipment for testing a microbe contained in a sample, **characterized by** comprising
an identification step of, when the inspection equipment irradiates the sample with a plurality of excitation light beams having different wavelengths, identifying a microbe contained in the sample on the basis of a distribution of peaks of fluorescence obtained from each object contained in the sample in correspondence with irradiation with the plurality of excitation light beams.

37. A computer-readable storage medium storing a control program which controls an inspection equipment for testing a microbe contained in a sample, **characterized in that** the control program comprises
an identification step of, when the inspection equipment irradiates the sample with a plurality of excitation light beams having different wavelengths, identifying a microbe contained in the sample on the basis of a distribution of peaks of fluorescence obtained from each object contained in the sample in correspondence with irradiation with the plurality of excitation light beams.
